# EUROPEAN PATENT APPLICATION

(11) **EP 2 638 896 A1**
(43) Date of publication of application: **18.09.2013**
(21) Application number: 12159497.2
(22) Date of filing: 14.03.2012
(51) Int. Cl.: A61K 9/127, A61P 35/02

(54) **Cationic liposomal drug delivery system for specific targeting of human cd14+ monocytes in whole blood**

(71) Applicant: Bioneer A/S, 2970 Hoersholm (DK); DTU Nanotech, 2800 Kongens Lyngby (DK)
(72) Inventor: Jensen, Simon Skjøde, 2700 Brønshøj (DK); Andresen, Thomas Lars, 2720 Vanløse (DK); Henriksen, Jonas Rosager, 3450 Allerød (DK); Johansen, Pia Thermann, 1954 Frederiksberg (DK)
(74) Representative: Inspicos A/S

(57) **Abstract**

This invention concerns a liposome comprising lipids and at least one active ingredient, wherein at least one of the lipids is a cationic lipid; said liposome exhibiting a net positive charge at physiological conditions at which said liposome preferentially adheres to monocytes in freshly drawn blood when compared to adherence to granulocytes, T-lymphocytes, B-lymphocytes and/or NK cells in freshly drawn blood, to a lipid-based pharmaceutical composition comprising said liposomes and their use in monocytic associated prophylaxis, treatment or amelioration of a condition such as cancer, an infectious disease, an inflammatory disease, an autoimmune disease or allergy.

## Description

### FIELD OF THE INVENTION

The present invention relates to specific delivery to monocytes using cationic liposomes. More particularly, the present invention relates to a liposome comprising lipids and at least one active ingredient, wherein at least one of the lipids is a cationic lipid; said liposome exhibiting a net positive charge at physiological conditions at which said liposome preferentially adheres to monocytes in freshly drawn blood, to a lipid-based pharmaceutical composition comprising said liposomes and their use in monocytic associated prophylaxis, treatment or amelioration.

### BACKGROUND OF THE INVENTION

Liposomes are lipid vesicles composed of a lipid bilayer membrane enclosing an aqueous core. These vesicles are considered to have great potential as drug delivery systems for several reasons; i) various types of drugs can be delivered; hydrophilic drugs can be loaded into the aqueous compartment or hydrophobic drugs can be anchored in the membrane, ii) the therapeutic efficacy is enhanced by targeting specific tissues resulting in increased bioavailability of the delivered drug, and iii) the side effects are significantly reduced, since only diseased tissues are exposed to the administered drugs. As a result liposomes have been studied extensively for the past decades in an attempt to develop novel formulations to treat e.g. cancer (1,2,3) and inflammation (4,5), but also to target specific tissues such as the brain, mitochondria, the ocular surface, and derma.

The uptake of liposomes by the mononuclear phagocyte system (MPS) after administration, which decreases the amount of drugs reaching the target site, was considered to be one of the major drawbacks of early liposomal drug delivery systems. The MPS consists of monocytes in the blood, their precursor cells in the bone marrow and tissue macrophages. Monocytes differentiate from hematopoietic stem cells in the bone marrow from where they are released into the blood. They can circulate for several days, before they as a result of pro-inflammatory, metabolic or immune stimuli leave the vasculature, migrate into the tissues and differentiate into macrophages or dendritic cells. Tissue macrophages especially found in the liver, spleen, and lymphatic system has high phagocytic activity and contributes to clearance of apoptotic cells, but also administrated liposomes. Monocytes in the blood also play an important role in elimination of pathogens and apoptotic host cells by phagocytosis. The phagocytic ability of cells of the MPS can be turned to an advantage by targeting the cells in the blood that normally unwanted take up the administered liposome formulations; the monocytes. Monocytes are the first cells to be recruited to the site of inflammation or infection, making monocytes important components of the first line of defense as well as in regulation of disease. Monocytes, macrophage and dendritic cells serve three main functions in the immune system; phagocytosis, antigen presentation and cytokine production. They play a central role in acute and chronic inflammation since they maintain the inflammatory condition by secretion of pro-inflammatory cytokines such as TNF-α, IL-1β and IL-6. Anti-inflammatory drugs given systemically have adverse side effects + localizes in healthy tissues or are rapidly excreted, a problem that can be circumvented by use of specific drug delivery systems. Therefore, targeted delivery to monocytes is of great importance.

Karathanasis *et al.* (6) have studied the uptake of a liposome formulation containing a specific positively charged peptide called GGP. They found, that they could target neutrophils and monocytes selectively, but also that it depended on the sequence of the peptide and not the positive charge, since a non targeting control peptide with same positive charge did not enhance uptake of liposomes. The charge of liposomes has however been reported to have significant effect on the cellular uptake of liposomes. Lee *et al.* (7) investigated the interaction of neutral vs. negatively charged liposomes on human monocytic cell linescultured in medium in presence of Fetal calf serum as suspension or adherent cells *in vitro.* They found, that uptake of negatively charged liposomes dependend on the maturation state of the monocytes. When the cells differentiated into macrophages the uptake of negatively charged liposomes increased 5-fold, whereas uptake of neutral liposomes stayed constant. The same did the uptake of both formulations in monocyte cell line cultures in suspension. The uptake of negatively charged liposomes by the human macrophage cell line J774 was also reported previously (8,9).

Cationic liposomes have been investigated in non-viral transfection systems due to their ability to condense RNA/DNA and due to the highly positive charge of the resulting lipoplexes they are generally internalized unselectively by many cells types (1, 2, 3, 10, 11, 12). Cationic peptides have been reported to be toxic to cells, but this effect can be circumvented by addition of co-lipids or co-polymers in the liposome formulation ().

US 6,120,799 discloses angiogenic endothelial cells selectively targeted with lipid/DNA complexes or cationic liposomes.

US2010/0068212 A1 relates to a method for targeting pathogenic monocytes.

US2002/0155609A1 discloses a monocyte-specific particulate delivery vehicle.

US2007/0292494A1 discloses carbohydrate-derivatized liposomes for targeting cellular carbohydrate recognition domains of CTL/CTLD lectins to deliver an active agent intracellularly to a reservoir cell that is infected with or susceptible to infection with an infectious agent, such as HIV.

US 2005/0142114 A1 relates to targeted lipid-drug formulations for delivery of drugs to myeloid and lymphoid immune cells.

US 2009/0232731 discloses cationic liposomal preparations for the treatment of rheumatoid arthritis.

Positively charged liposomes may exhibit cytotoxic activity ( Lv J Control Release. 2006 Aug 10;114(1):100-9, and C. Kelly et al., J Drug Deliv. 2011:article ID 727241).

Cationic liposomes are used for delivery of nucleic acids in e.g. gene delivery technologies or oligo nucleotide based knock out technologies (14).

If a liposomal drug delivery system is to be used for immune modulation the formulation must be taken up by monocytes, but not by other cell populations in the blood. The present invention provides a liposomal formulation that is taken up selectively by monocytes in fresh whole blood. To accomplish this, the interactions between various liposome formulations and the specific cell populations in the blood were investigated.

### SUMMARY OF THE INVENTION

Surprisingly, it has been found that liposomes with a net positive charge at physiological conditions are able to target monocytes selectively when incubated in fresh whole blood.

According to a first aspect, the invention concerns a liposome for monocyte targeting comprising lipids and at least one active ingredient, wherein at least one of the lipids is a cationic lipid; said liposome exhibiting a net positive charge at physiological conditions at which said liposome preferentially adheres to monocytes in freshly drawn blood when compared to adherence to granulocytes, T-lymphocytes, B-lymphocytes and/or NK cells in freshly drawn blood, thereby allowing selective targeting and association with monocytes present in the blood or tissue.

Positively charged liposomes exhibit a superior association with and retention to monocytes either by cell-membrane association, phagocytosis or endocytosis. These mechanisms may be induced by the complement system which is active in fresh human blood, but which is inactivated during storage or freezing of blood, plasma and serum. Several complement factors show biological half lives in the range of minutes.

According to second aspect, the invention concerns a lipid-based delivery system for targeting monocytes in fresh blood, said system providing delivery to and release of at least one active ingredient to the targeted monocyte, said system comprising:
(I) lipids comprising:
   at least one cationic lipid; and
(II) at least one active ingredient;
said lipids allowing the formation of liposomes, said liposomes comprising at least part of said at least one active ingredient; said liposome exhibiting a net positive charge at physiological conditions at which said liposome preferentially adheres to monocytes in freshly drawn blood when compared to adherence to granulocytes, T-lymphocytes, B-lymphocytes and/or NK cells in freshly drawn blood.

According to third aspect, the invention concerns a pharmaceutical formulation comprising the liposome according to the first aspect of the invention.

The person skilled in the art is aware of suitable excipients to include in such a pharmaceutical formulation. However, reference is made to the detailed disclosure below.

According to another aspect, the invention concerns a liposome according to the first aspect of the invention, the lipid delivery system according to the second aspect of the invention or a pharmaceutical composition according to the third aspect of the invention for use as a pharmaceutical.

The liposome, the lipid delivery system or the pharmaceutical composition according to the invention may be used as a pharmaceutical for the prophylaxis, treatment or amelioration of a number of conditions as disclosed in more detail below.

According to another aspect, the invention concerns a liposome according to the first aspect of the invention, the lipid delivery system according to the second aspect of the invention or a pharmaceutical composition according to the third aspect of the invention for use in a monocytic associated prophylaxis, treatment or amelioration.

Thus by targeting the monocytes, which play a central role in the immune system it has become possible to tailor the drug delivery to the desired site of action.

According to another aspect, the invention concerns a use of the liposome according to the first aspect of the invention, the lipid delivery system according to the second aspect of the invention or a pharmaceutical composition according to the third aspect of the invention for the preparation of a medicament for use in a monocytic associated prophylaxis, treatment or amelioration.

According to another aspect, the invention concerns a method for in vitro activation/inhibition of monocytes, comprising the steps:
i) Providing fresh blood;
ii) Administering a liposome according to any one of claims 1-28, the lipid delivery system according to claim 29 or the pharmaceutical composition according to claim 30 to said fresh blood;
iii) Allowing said liposome, lipid delivery system or pharmaceutical composition to react.

Thereby it will be possible to analyse the monocyte fraction for immune stimulation or immune suppression of monocyte function.

According to another aspect, the invention concerns a method for in vivo activation/inhibition of monocytes in a mammal, comprising the step of administering a liposome according to the first aspect of the invention, the lipid delivery system according to the second aspect of the invention or a pharmaceutical composition according to the third aspect of the invention to said mammal in an amount sufficient to activate/inhibit said monocytes.

According to another aspect, the invention concerns a method for in vivo activation/inhibition of monocytes in a mammal, comprising the steps:
i) Providing fresh blood from a mammal in need thereof;
ii) Administering a liposome according to the first aspect of the invention, the lipid delivery system according to the second aspect of the invention or a pharmaceutical composition according to the third aspect of the invention to said fresh blood;
iii) Allowing said liposome, lipid delivery system or pharmaceutical composition to react with said fresh blood;
iv) Reintroducing said blood into the circulation of said mammal.

Thereby inducing or suppressing immune functions through monocytes in said mammal without directly administering the lipid delivery system or pharmaceutical to that mammal.

According to another aspect, the invention concerns a method for prophylactic or therapeutic treatment or amelioration of cancer, an infectious disease, an inflammatory disease, an autoimmune disease or allergy, the method comprising administering to a subject in need thereof an effective amount of a liposome according to the first aspect of the invention, the lipid delivery system according to the second aspect of the invention or a pharmaceutical composition according to the third aspect of the invention.

### LEGENDS TO THE FIGURE

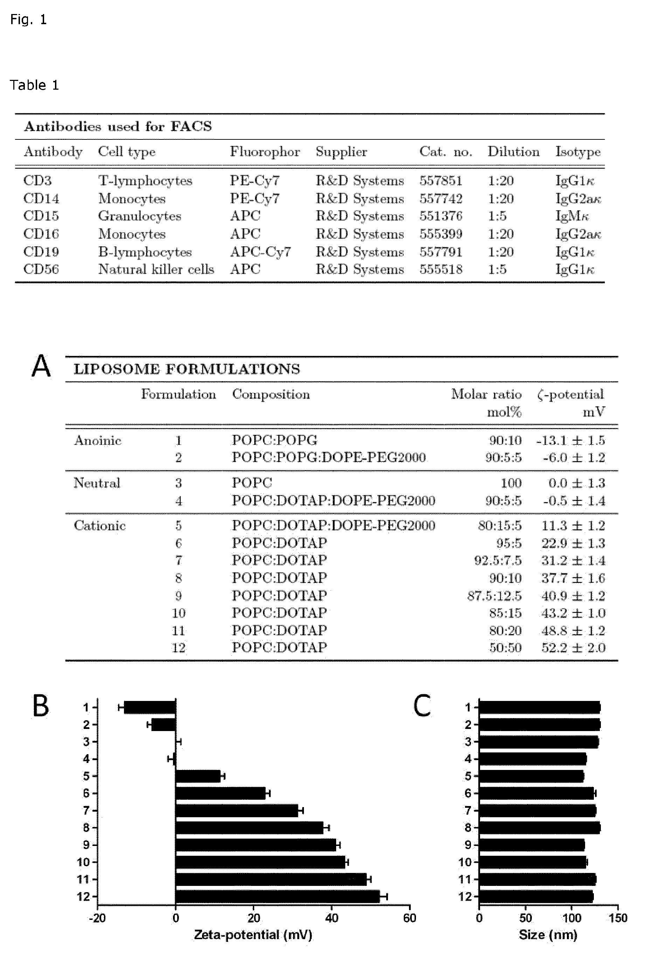

Fig. 1: Overview of liposomes, liposome size and surface charge. (A) List of liposomes composed of 1-palmitoyl-2-oleoyl-sn-glycero-3-phosphocholine (POPC), 1-palmitoyl-2-oleoyl-sn-glycero-3-phosphoglycerol (POPG) and 1,2- dioleoyl -sn-glycero-3-phosphoethanolamine-N-[methoxy(polyethylene glycol)-2000] (DOPE-PEG2000). Ratio of each component in the liposomes is shown. (B) Liposome surface charge expressed in mV. (C) Liposome size measured in nanometer (nm). Liposome compositions for B and C refer to the numbers from figure 1A.

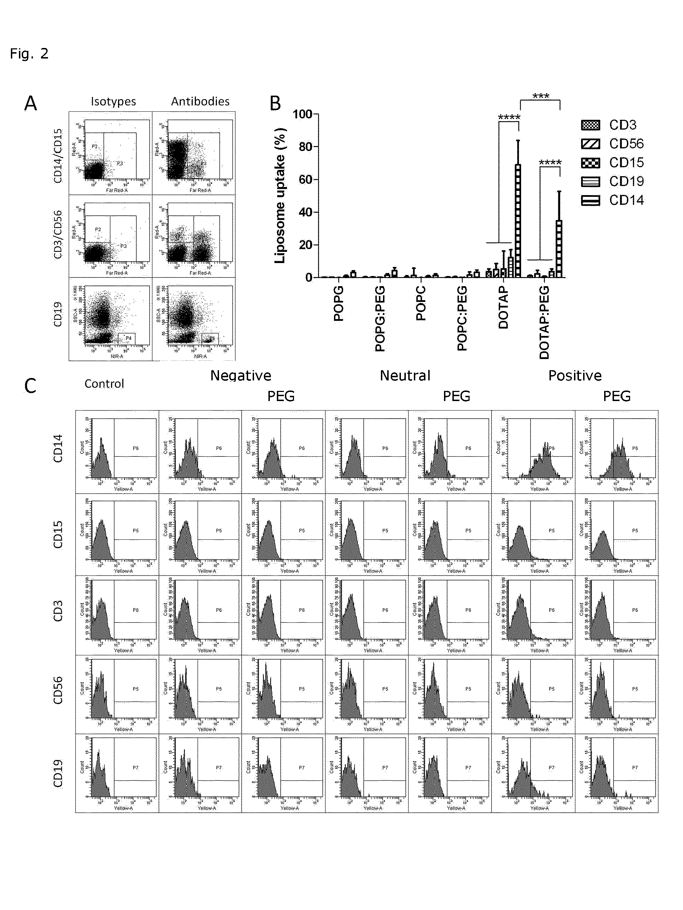

Fig. 2: Uptake of liposome formulations containing POPC (formulation 3 and 4) with or without addition of negative (POPG, formulation 1 and 2) or positive (DOTAP, formulation 5 and 8) lipids. (A) Antibodies used as markers for five different cell populations in whole blood samples; CD14 (monocytic marker), CD15 (granulocytic marker), CD3 (T-lymphocytic marker), CD19 (B-lymphocytic marker), and CD56 (NK cell marker). (B) FACS analysis of the uptake of six different liposome formulations containing RhB (compositions 1-4, 8 and 5, respectively, in Fig. 1A)(POPC: POPG 90:10, POPC: POPG: DOPE-PEG2000 90:5:5, POPC, POPC: DOTAP: DOPE-PEG2000 90: 5: 5, POPC: DOTAP 90:10, POPC: DOTAP: DOPE-PEG2000 80:15:5). The data are expressed as percentages of cells positive for RhB shown as mean ± SD. Each group is analyzed by one-way ANOVA. When two values are compared a two-tailed t-test is used (*** P<0.001, **** P<0.0001). (C) Representative graphs showing gating of positive cells in the 5 cell populations.

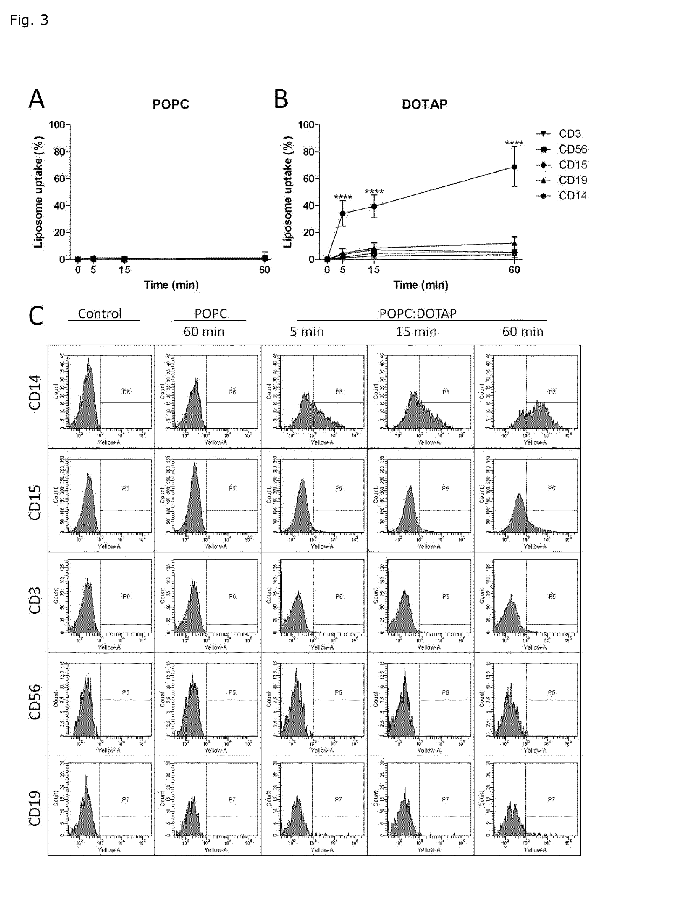

Fig. 3: Uptake of liposome formulation 3 (A) and 8 (B) after 0, 5, 15 and 60 min incubation are shown. Data are expressed as percentages of cells positive for RhB shown as mean ± SD. Each group is analyzed by one-way ANOVA (**** P<0.0001). (C) Representative graphs showing gating of positive cells in the 5 cell populations.

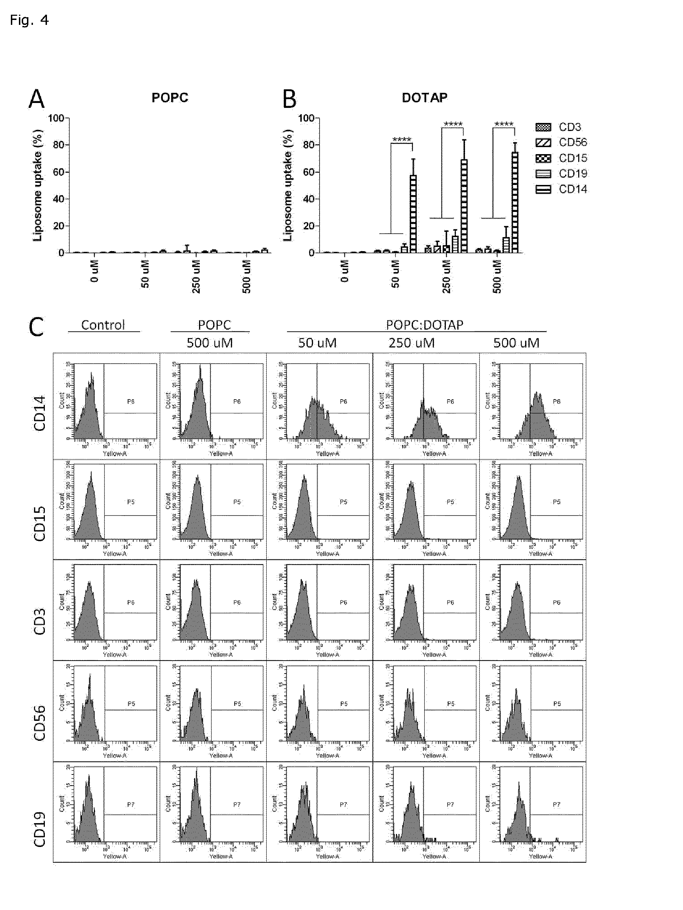

Fig. 4: Uptake of liposome formulation 3 (A) and 8 (B) containing RhB (Fig. 1A) at a concentration of 0, 50, 250, and 500mM. Data are expressed as percentages of cells positive for RhB shown as mean ± SD. Each group is analyzed by one-way ANOVA (**** P<0.0001). (C) Representative graphs showing gating of positive cells in the 5 cell populations.

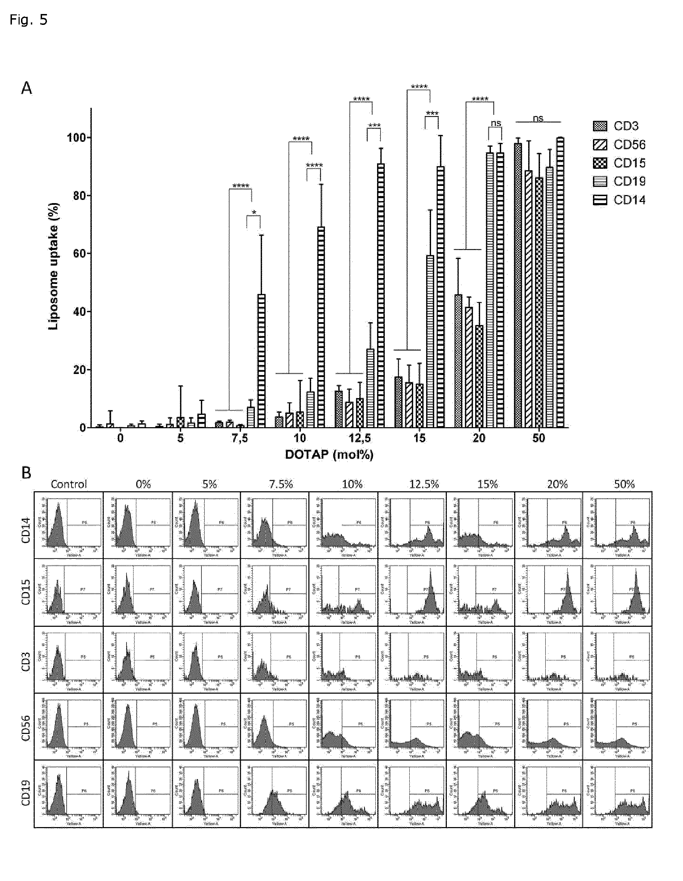

Fig. 5: The effect of positive charge on uptake in the different cell populations was analyzed. (A) Uptake of liposome formulations containing 0-50 mol% DOTAP (composition 3 and 6-12 in Fig. 1A). Data are expressed as percentages of cells positive for RhB shown as mean ± SD. Each group is analyzed by one-way ANOVA. When two values are compared a two-tailed t-test is used (*** P<0.001, **** P<0.0001, ns = not significant). (B) Representative graphs showing gating of positive cells in the 5 cell populations.

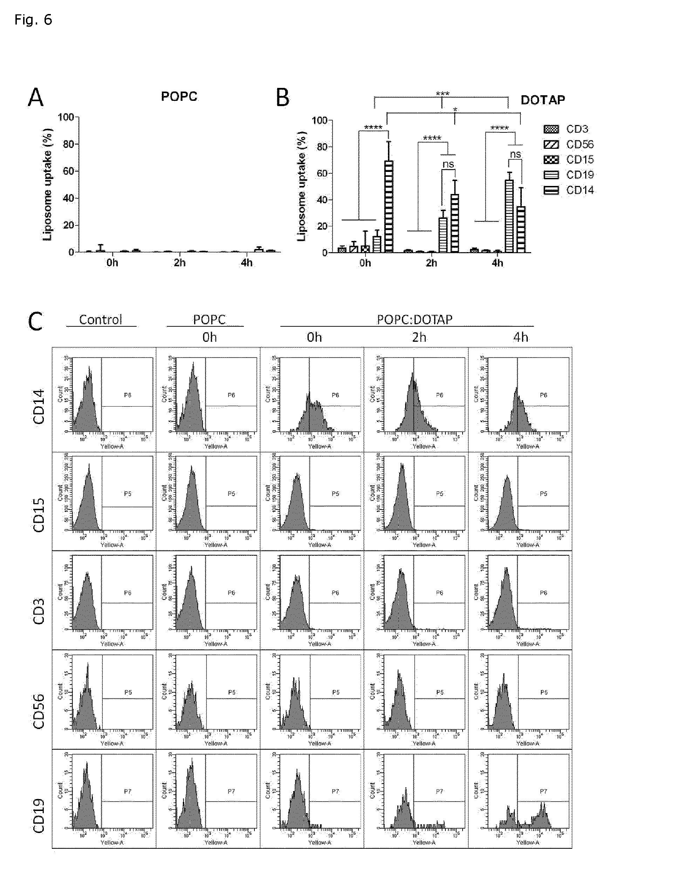

Fig. 6: Uptake of liposome formulation 3 (A) and 8 (B) containing RhB (Fig. 1A) after 0, 2h and 4h incubation of the blood at 37C with rotation are shown. Data are expressed as percentages of cells positive for RhB shown as mean ± SD. Each group is analyzed by one-way ANOVA. When two values are compared a two-tailed t-test is used (* P<0.05, *** P<0.001, **** P<0.0001). (C) Representative graphs showing gating of positive cells in the 5 cell populations.

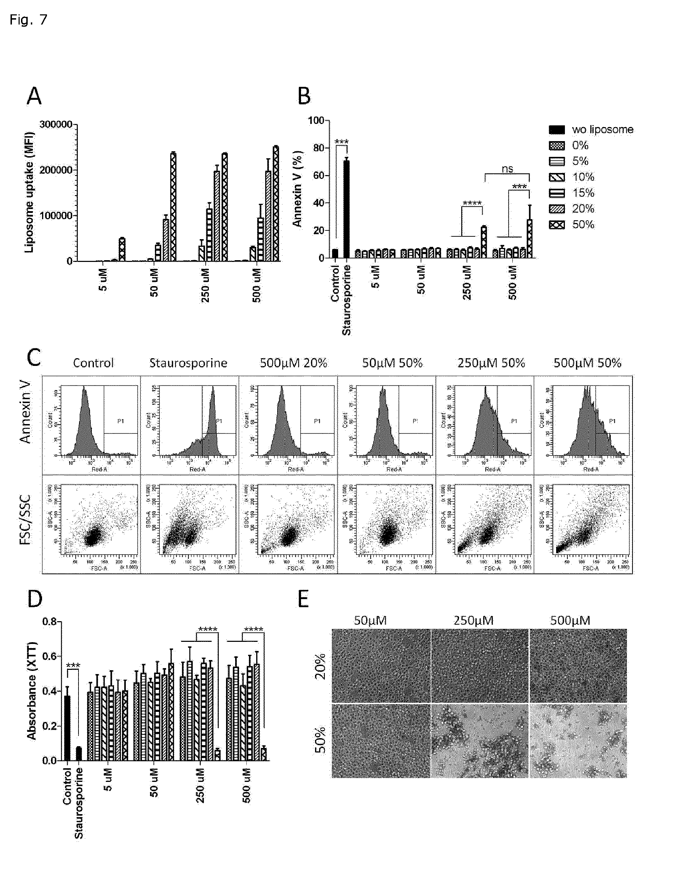

Fig. 7: Liposome cytotoxicity was measured on the human monocytic cell line THP-1 using liposomes containing 0- 50% DOTAP (composition 3 and 6-12 in Fig. 1A). Four different concentrations of liposome were used: 5, 50, 250 and 500 µM. (A) FACS analysis of uptake of liposomes in THP-1 cells after 60 min incubation. (B) Liposomes were incubated with cells for 60 min before washing with cell media. After 24 h annexin V binding to the cells was measured by FACS. Staurosporine is used as positive control for apoptosis. (C) Representative graphs showing gating of annexin V positive cells. (D) Liposomes were incubated with cells for 60 min before washing with cell media. After 24 h the liposome induced cytotoxicity was measured by incubation with XTT for 4 hours. (E) Phase contrast microscopy pictures showing cells after XTT incubation. Data are expressed as MFI of liposome uptake (A), percentages of cells positive for annexin V (B) or absorbance (D) shown as mean ± SD. Each group is analyzed by one-way ANOVA. When two values are compared a two-tailed t-test is used (*** P<0.001, **** P<0.0001, ns = not significant).

### DETAILED DISCLOSURE OF THE INVENTION

### Definitions

A "liposome" in the present application and claims denotes an artificial prepared vesicle made of at least one lipid bilayer.

The term "preferentially adheres" as used in the present context means that the liposomes according to the invention adheres to monocytes in freshly drawn blood to an extent which is at least 1.5 times larger than the adherence to granulocytes, T-lymphocytes, B-lymphocytes and/or NK cells in freshly drawn blood, preferably at least 2 times, such as at least 3 times, more preferably at least 4 times, such as at least 5 times larger than the adherence to granulocytes, T-lymphocytes, B-lymphocytes and/or NK cells in freshly drawn blood .

The term "freshly drawn blood" in the present context means that the blood in question has been drawn from a mammal within no more than 60 minutes, such as no more than 30 minutes, preferably no more than 15 minutes, no more than 10 minutes, such as no more than 5 minutes.

The term "zeta potential" in the present context describes the electric potential at the location of the slipping plane of a colloidal particle in solution versus a point in the bulk fluid away from the interface. The term "relative zeta potential" in the present context for a liposome X is defined as (zeta potential of X minus zeta potential of liposome 6 of fig. 1 (5% DOTAP)) divided by (difference between zeta potential of liposome 11 of fig. 1 (20% DOTAP) minus zeta potential of liposome 6 of fig. 1 (5% DOTAP))x 100; measured in 10% sucrose buffer under the same conditions as disclosed in detail in ex. 2.

The term "immunogenic" means capable of inducing an immunological response.

The term "tolerogenic" as used in the present context means capable of down-modulating an immunological response.

When using the terms "substantial"/"substantially" or "essential"/"essentially" herein it is intended that the feature which is described by these terms is present in an amount or has an impact which provides for a technical effect with relevance for the exercise of the presently claimed invention. For instance, a "substantial amount" of a substance in a composition is an amount which provides for a technical effect exhibited by the substance to a degree which provides for a technical effect in terms of the present invention. Likewise, if a composition is indicated as comprising "substantially no" of a particular substance, this means that the composition is allowed to include insignificant amounts of the substance, as long as these amounts do not have any technical impact on the other ingredients in the composition and does not in itself "make a difference" - or put in other words, "substantially no" and "essentially no" means that e.g. trace amounts or effects may be present as long as they do not have an overall technical influence.

### Specific embodiments of the invention

Additional embodiments of the present invention are described below. It will be clear for the person skilled in the art, that aspects and/or embodiments of the invention may be combined.

An embodiment of the invention is a liposome having a relative zeta potential of between 15 and 85%.

As it appears from the results reported herein, the liposome according to the invention, at a relative zeta potential in the range 15-85% selectively adheres to monocytes.

An embodiment of the invention is a liposome, wherein said liposomes are suitable for intravenous administration.

An embodiment of the invention is a liposome having a relative zeta potential of between 20 and 80%.

An embodiment of the invention is a liposome having a relative zeta potential of between 25 and 75%.

More particularly, the relative zeta potential is in the range 20-80%, even more particularly in the range 25-75%. Within these ranges the selectivity for monocytes compared to other blood components such as granulocytes, T-lymphocytes, B-lymphocytes and/or NK cells is even more pronounced.

An embodiment of the invention is a liposome, wherein the liposomes comprise less than 20%, such as less than 10%, preferably less than 5%, more preferred less than 2% cholesterol, most preferred substantially no cholesterol.

### Liposome structure and characteristics

An embodiment of the invention is a liposome, wherein at least part of the lipids are selected from the group consisting of phospholipids, sterols and sterol derivatives.

A particular embodiment of the invention is a liposome, wherein the lipid comprises or constitutes a member selected from the group consisting of phosphatidylcholine (PC), phosphatidylethanolamine (PE), phosphatidylserine (PS), phosphatidylglycerol (PG), phosphatidylinositol (PI), phosphatidic acid (PA), DPG (bisphosphatidyl glycerol), PEOH (phosphatidyl alcohol), cholesterol, ergosterol and lanosterol.

An embodiment of the invention is a liposome, wherein the phosphatidylcholines are selected from the group consisting of 1,2-dioleoyl-phosphatidylcholine, 1,2-dipalmitoyl-phosphatidylcholine, 1,2-dimyristoyl-phosphatidylcholine, 1,2-distearoyl-phosphatidylcholine, 1-oleoyl-2-palmitoyl-phosphatidylcholine, 1-oleoyl-2-stearoyl-phosphatidylcholine, 1-palmitoyl-2-oleoyl-phosphatidylcholine and 1-stearoyl-2-oleoyl-phosphatidylcholine.

An embodiment of the invention is a liposome, wherein the phosphatidylcholines are selected from the group consisting of 1,2-dioleoyl-phosphatidylcholine, 1,2-dipalmitoyl-phosphatidylcholine, 1,2-dimyristoyl-phosphatidylcholine, 1,2-distearoyl-phosphatidylcholine, and 1-palmitoyl-2-oleoyl-phosphatidylcholine.

A particular embodiment of the invention is a liposome, wherein the lipid comprises 1,2-dioleoyl-phosphatidylcholine.

Another embodiment of the invention is a liposome, wherein the phosphatidylethanolamines are selected from the group consisting of 1,2-dioleoyl-phosphatidylethanolamine, 1,2-dipalmitoyl-phosphatidylethanolamine, 1,2-dimyristoyl-phosphatidylethanolamine, 1,2-distearoyl-phosphatidylethanolamine, 1-oleoyl-2-palmitoyl-phosphatidylethanolamine, 1-oleoyl-2-stearoyl-phosphatidylethanolamine, 1-palmitoyl-2-oleoyl-phosphatidylethanolamine, 1-stearoyl-2-oleoyl-phosphatidylethanolamine and N-succinyl-dioleoyl-phosphatidylethanolamine; the phosphatidylserines are selected from the group consisting 1,2-dioleoyl-phosphatidylserine, 1,2-dipalmitoyl-phosphatidylserine, 1,2-dimyristoyl-phosphatidylserine, 1,2-distearoyl-phosphatidylserine, 1-oleoyl-2-palmitoyl-phosphatidylserine, 1-oleoyl-2-stearoyl-phosphatidylserine, 1-palmitoyl-2-oleoyl-phosphatidylserine and 1-stearoyl-2-oleoyl-phosphatidylserine; the phosphatidylglycerols are selected from the group consisting 1,2-dioleoyl-phosphatidylglycerol, 1,2-dipalmitoyl-phosphatidylglycerol, 1,2-dimyristoyl-phosphatidylglycerol, 1,2-distearoyl-phosphatidylglycerol, 1-oleoyl-2-palmitoyl-phosphatidylglycerol, 1-oleoyl-2-stearoyl-phosphatidylglycerol, 1-palmitoyl-2-oleoyl-phosphatidylglycerol and 1-stearoyl-2-oleoyl-phosphatidylglycerol; the phosphatidic acids are selected from the group consisting of di-palmitoyl-glycerophosphatidic acid, di-stearoyl-glycerophosphatidic acid, di-myrostoyl-glycerophosphatidic acid, di-oleoyl-glycerophosphatidic acid, palmitoyl-oleoyl-glycerophosphatidic acid.

Another embodiment of the invention is a liposome, wherein the lipid comprises or constitutes phosphatidylglycerol (PG), phosphatidylethanolamine (PE), phosphatidylserine (PS), phosphatidylcholine (PC), phosphatidylinositol (PI), phosphatidic acid (PA), DPG (bisphosphatidyl glycerol), PEOH (phosphatidyl alcohol),cholesterol, phosphatidylcholines such as 1,2-dioleoyl-phosphatidylcholine, 1,2-dipalmitoyl-phosphatidylcholine, 1,2-dimyristoyl-phosphatidylcholine, 1,2-distearoyl-phosphatidylcholine, 1-oleoyl-2-palmitoyl-phosphatidylcholine, 1-oleoyl-2-stearoyl-phosphatidylcholine, 1-palmitoyl-2-oleoyl-phosphatidylcholine and 1-stearoyl-2-oleoyl-phosphatidylcholine; phosphatidylethanolamines such as 1,2-dioleoyl-phosphatidylethanolamine, 1,2-dipalmitoyl-phosphatidylethanolamine, 1,2-dimyristoyl-phosphatidylethanolamine, 1,2-distearoyl-phosphatidylethanolamine, 1-oleoyl-2-palmitoyl-phosphatidylethanolamine, 1-oleoyl-2-stearoyl-phosphatidylethanolamine, 1-palmitoyl-2-oleoyl-phosphatidylethanolamine, 1-stearoyl-2-oleoyl-phosphatidylethanolamine and N-succinyl-dioleoyl-phosphatidylethanolamine; phosphatidylserines such as 1,2-dioleoyl-phosphatidylserine, 1,2-dipalmitoyl-phosphatidylserine, 1,2-dimyristoyl-phosphatidylserine, 1,2-distearoyl-phosphatidylserine, 1-oleoyl-2-palmitoyl-phosphatidylserine, 1-oleoyl-2-stearoyl-phosphatidylserine, 1-palmitoyl-2-oleoyl-phosphatidylserine and 1-stearoyl-2-oleoyl-phosphatidylserine; phosphatidylglycerols such as 1,2-dioleoyl-phosphatidylglycerol, 1,2-dipalmitoyl-phosphatidylglycerol, 1,2-dimyristoyl-phosphatidylglycerol, 1,2-distearoyl-phosphatidylglycerol, 1-oleoyl-2-palmitoyl-phosphatidylglycerol, 1-oleoyl-2-stearoyl-phosphatidylglycerol, 1-palmitoyl-2-oleoyl-phosphatidylglycerol and 1-stearoyl-2-oleoyl-phosphatidylglycerol; 1,2-dioctadecanoyl-sn-glycero-3-ethylphosphocholine (Ethyl PC); pegylated lipids; pegylated phospoholipids such as phophatidylethanolamine-N-[methoxy(polyethyleneglycol)-1000], phophatidylethanolamine-N-[methoxy(polyethyleneglycol)-2000], phophatidylethanolamine-N-[methoxy(polyethylene glycol)-3000], phophatidylethanolamine-N-[methoxy(polyethyleneglycol)-5000]; pegylated ceramides such as N-octanoyl-sphingosine-1-{succinyl[methoxy(polyethyleneglycol)1000]}, N-octanoyl-sphingosine-1-{succinyl[methoxy(polyethylene glycol)2000]}, N-octanoyl-sphingosine-1-{succinyl[methoxy(polyethyleneglycol)3000]}, N-octanoyl-sphingosine-1-{succinyl[methoxy(polyethyleneglycol)5000]};lyso-phosphatidylcholines, lyso-phosphatidylethanolamines, lyso-phosphatidylglycerols, lyso-phosphatidylserines, ceramides; sphingolipids; glycolipids such as ganglioside GMI; glucolipids; sulphatides; phosphatidic acid, such as di-palmitoyl-glycerophosphatidic acid; palmitic fatty acids; stearic fatty acids; arachidonic fatty acids; lauric fatty acids; myristic fatty acids; lauroleic fatty acids; physeteric fatty acids; myristoleic fatty acids; palmitoleic fatty acids; petroselinic fatty acids; oleic fatty acids; isolauric fatty acids; isomyristic fatty acids; isostearic fatty acids; sterol and sterol derivatives such as cholesterol, cholesterol hemisuccinate, cholesterol sulphate, and cholesteryl-(4-trimethylammonio)-butanoate, ergosterol, lanosterol; polyoxyethylene fatty acids esters and polyoxyethylene fatty acids alcohols; polyoxyethylene fatty acids alcohol ethers; polyoxyethylated sorbitan fatty acid esters, glycerol polyethylene glycol oxy-stearate; glycerol polyethylene glycol ricinoleate; ethoxylated soybean sterols; ethoxylated castor oil; polyoxyethylene polyoxypropylene fatty acid polymers; polyoxyethylene fatty acid stearates; di-oleoyl-sn-glycerol; dipalmitoyl-succinylglycerol; 1,3-dipalmitoyl-2-succinylglycerol;1-alkyl-2-acyl-phosphatidylcholines such as 1-hexadecyl-2-palmitoyl-phosphatidylcholine; 1-alkyl-2-acyl-phosphatidylethanolamines such as 1-hexadecyl-2-palmitoyl-phosphatidylethanolamine; 1-alkyl-2-acyl-phosphatidylserines such as 1-hexadecyl-2-palmitoyl-phosphatidylserine; 1-alkyl-2-acyl-phosphatidylglycerols such as 1-hexadecyl-2-palmitoyl-phosphatidylglycerol; 1-alkyl-2-alkyl-phosphatidylcholines such as 1-hexadecyl-2-hexadecyl-phosphatidylcholine; 1-alkyl-2-alkyl-phosphatidylethanolamines such as 1-hexadecyl-2-hexadecyl-phosphatidylethanolamine; 1-alkyl-2-alkyl-phosphatidylserines such as 1-hexadecyl-2-hexadecyl-phosphatidylserine; 1-alkyl-2-alkyl-phosphatidylglycerols such as 1-hexadecyl-2-hexadecyl-phosphatidylglycerol; and N-Succinyl-dioctadecylamine; palmitoylhomocysteine.

An embodiment of the invention is a liposome, wherein at least part of the lipids is a cationic lipid.

It has thus been found that liposomes having a net positive charge adhere preferentially to monocytes compared to adherence to granulocytes, T-lymphocytes, B-lymphocytes and/or NK cells.

An embodiment of the invention is a liposome, wherein the cationic lipids are selected from the group consisting of stearylamine (SA), lauryltrimethylammonium bromide; cetyltrimethylammonium bromide, myristyl trimethylammonium bromide, dimethyldioctadecylammonium bromide (DDAB), 3β-[N-(N',N'-dimethylaminoethane)-carbamoyl]cholesterol (DC-Cholesterol), 1,2-ditetradecanoyl-3-trimethylammonium-propane (DMTAP), 1,2-dioctadecanoyl-3-trimethylammonium-propane (DOTAP) and DOTAP derivatives such as 1,2-di-(9Z-octadecenoyl)-3-trimethylammonium-propane and 1,2-dihexadecanoyl-3-trimethylammonium-propane, 1,2-di-(9Z-octadecenoyl)-3-dimethylammonium-propane (DODAP) and DODAP derivatives such as 1,2-ditetradecanoyl-3-dimethylammonium-propane, 1,2-dihexadecanoyl-3-dimethylammonium-propane, and 1,2-dioctadecanoyl-3-dimethylammonium-propane, 1,2-di-O-octadecenyl-3-trimethylammonium propane (DOTMA), 1,2-dioleoyl-c-(4'-trimethylammonium)-butanoyl-sn-glycerol (DOTB), dioctadecylamide-glycylspermine, SAINT-2, polycationic lipid 2,3-dioleyloxy-N-[2(spermine-carboxamido)ethyl]-N,N-dimethyl-1-propanaminiumtrifluoroacetate (DOSPA), and GL67TM.

A particular embodiment of the invention is a liposome, wherein the cationic lipids are selected from the group consisting of stearylamine (SA), 1,2-dioctadecanoyl-3-trimethylammonium-propane (DOTAP) and 1,2-di-(9Z-octadecenoyl)-3-dimethylammonium-propane (DODAP), preferably 1,2-dioctadecanoyl-3-trimethylammonium-propane (DOTAP).

Preferred cationic lipids are DOTAP and DOTAP derivatives. Additional examples of cationic lipids and lipid components may be found in or made according to US 4,804,539.

An embodiment of the invention is a liposome, wherein at least part of the lipids is a cationic lipopeptide selected from the group consisting of a lipid polyarginine conjugate, a lipid TAT conjugate, a lipid polylysine conjugate, or a cationic liposaccharide or lipopolysaccharide such as a lipid chitosan conjugate.

An embodiment of the invention is a liposome, wherein the liposomes comprise 0.5-50%, preferably 1-20%, such as 3-15, such as 7.5-12.5% (mol/mol) cationic lipids.

As it appears from the results reported herein, at ranges of cationic lipids in the above range selectivity for monocytes is obtained.

An embodiment of the invention is a liposome, which, when relevant, contains alkyl chains of the lipids that are C8-C24, preferably C10-C22, more preferred C12-C20, preferably C14-C18, most preferred C16-C18 saturated chains or unsaturated chains, preferably saturated chains.

An embodiment of the invention is a liposome, wherein at least one liposome is a Large Unilamellar Vesicle (LUV).

Preferred liposomes of the invention are in the form of Large Unilamellar Vesicles (LUV), meaning that LUVs are preferred components of a pharmaceutical composition comprising liposomes of the invention.

An embodiment of the invention is a liposome, wherein the liposomes have a diameter of 40-2000 nm, preferably 80-1000 nm, more preferred 100-500 nm, preferred 50-200 nm, more preferred 100-150 nm, preferably 100-400 nm.

The liposome of the invention includes an active ingredient, typically a drug substance or composition. Typically, the liposome comprises substantially all of said at least one active ingredient. Normally, one of the compartments selected among the interior aqueous compartment, a hydrophobic bilayer, and a polar inter-phase of the inner and outer leaflet of the liposome carry said at least one active ingredient.

The skilled person will generally be knowledgeable about the choice of active ingredient and the correct dosage thereof.

In preferred embodiments, the active ingredient is in the form of an immuno stimulating compound, preferably with the ability to stimulate the innate immune system.

An embodiment of the invention is thus a liposome, wherein said at least one active ingredient is an immuno stimulating compound which is a ligand for intracellular proteins and /or receptors.

An embodiment of the invention is a liposome, wherein said intracellular proteins and /or receptors are selected from the group consisting of TLR3, TLR7, TLR8, TLR9 , NOD1, NOD2, NOD5, NALP1, NALP2, NALP3, NALP12, NALP14, IPAF, NAIP, CIITA, RIG-I, MDA5, and LGP2, preferably selected from TLR3, TLR7, TLR8, TLR9 , and NOD2, more preferably TLR7.

An embodiment of the invention is a liposome, wherein said at least one active ingredient is an immuno stimulating compound selected from the group consisting of polyinosinic:polycytidylic acid (poly I:C), Polyadenylic-polyuridylic acid (poly A:U), poly I:C-poly-L-lysine (poly-ICLC), poly-ICR, CL264, *N*-palmitoyl-*S*-[2,3-bis(palmitoyloxy)-(2*R,S*)-propyl]- (R)-cysteine-(S)serine-(S)lysine 4 (Pam₃Cys), Monophosphoryl lipid A (MPLA) and other lipopolysaccharides, alpha-galactosylceremaide (aGC), Propirimine, Imiquimod (R837), resiquimod (R848), Gardiquimod, R850 (3M Pharma), R851 (3M Pharma), 852A (3M Pharma), S-27610, 3M-002 (CL075), 3M-003, 3M-005, 3M-006, 3M-007, 3M-012, 3M-13, 3M-031,3M-854 (3M Pharma), CL097, CL264, IC-31, Loxoribine and other imidazoquinolines, ssPolyU, sotirimod (3M Pharma), Isatoribine (Anadys), ANA975 (Anadys/Novartis), SM360320 (Sumitomo), R1354 (Coley Pharmaceuticals) single stranded or double stranded RNA, ORN 02 (5'-UUAUUAUUAUUAUUAUUAUU-3'), ORN 06 5'-UUGUUGUUGUUGUUGUUGUU-3', CpG-ODN DSLIM (Mologen), AVE 0675 (Coley Pharmaceuticals), CpG B oligodeoxynucleotide (ODN) 1018 (Dynavax Technologies ), AZD 1419 (Dynavax), ODN 1982, CpG B ODN 2006

(Coley Pharmaceuticals), IMO 2125 (Idera Pharma), CpG A ODN 2216, CpG A ODN 2336, CpG 2395, CpG ODN 7909 (Coley Pharmaceuticals), CpG 10101 (Coley Pharmaceuticals), CpG ODN AVE0675 (Coley Pharmaceuticals), CpG ODN HYB2093 (Idera Pharmaceuticals/Novartis), CpG ODN HYB2055 (Idera Pharmaceuticals), CpG-ODN IMO-2125 (Idera Pharmaceuticals), CpG C ODN M362, Tolamba (Amb a1 ragweed allergen with covalently linked CpG B class ODN 1018)(Dynavax Technologies), Heplisav (Dynavax Technologies), 10181SS (Dynavax Technologies), IM02055 (Idera Pharmaceuticals), IRS954 (Dynavax Technologies), (flagellin, muramyl dipeptide, saponins such as QS21, Leishmania elongation factor, SB-AS4, threonyl-muramyl dipeptide, L18-MDP, mifamurtid, and OM-174. An embodiment of the invention is a liposome, wherein said at least one active ingredient is an immuno stimulating compound selected from the group consisting of Monophosphoryl lipid A (MPLA), Imiquimod (R837), resiquimod (R848), Gardiquimod, Loxoribine, sotirimod (3M Pharma), Isatoribine (Anadys), SM360320 (Sumitomo), CpG B oligodeoxynucleotide (ODN) 1018 (Dynavax Technologies ), AZD 1419 (Dynavax), ODN 1982, CpG B ODN 2006 (Coley Pharmaceuticals), IMO 2125 (Idera Pharma), CpG A ODN 2216, CpG A ODN 2336, CpG 2395, CpG ODN 7909 (Coley Pharmaceuticals), CpG 10101 (Coley Pharmaceuticals), CpG ODN AVE0675 (Coley Pharmaceuticals), CpG ODN HYB2093 (Idera Pharmaceuticals/Novartis), CpG ODN HYB2055 (Idera Pharmaceuticals), CpG-ODN IMO-2125 (Idera Pharmaceuticals), CpG C ODN M362, Tolamba (Amb a1 ragweed allergen with covalently linked CpG B class ODN 1018)(Dynavax Technologies), Heplisav (Dynavax Technologies), QS21, L18-MDP, mifamurtid, and OM-174.

An embodiment of the invention is a liposome, wherein said at least one active ingredient is an immuno stimulating compound selected from the group consisting of Mifamurtid (2-[(*N-*{(2*R*)-[(2-acetamido-2,3-dideoxy-D-glucopyranos-3-yl)oxy]-propanoyl}-L-alanyl-D-isoglutaminyl-L-alanyl)amino]ethyl (2*R*)-2,3-bis(hexadecanoyloxy)propyl hydrogen phosphate), L18MDP (6-O-stearoyl-N-Acetyl-muramyl-L-alanyl-D-isoglutamine), as well as acylated or C₁₂₋₂₀ saturated or unsaturated, ester or ether bound derivatives of the above mentioned immuno stimulatory compounds.

An embodiment of the invention is a liposome, wherein said at least one active ingredient is an immuno suppressive compound comprising a steroid selected from the group consisting of 21-Acetoxyprefnenolone, Aalclometasone, Algestone, Amicinonide, Beclomethasone, Betamethasone, Betamethasone dipropionate, Betamethasone hemisuccinate, Budesonide, Chloroprednisone, Clobetasol, Blovetasone, Clocortolone, Cloprednol, Corticosterone, Cortisone, Cortivazol, Deflazacort, Desonide, Desoximethasone, dexamethason, Dexamethasone palmitate, Dexamethasone phosphate, Diflorasone, Diflucortolone, Difluprednate, Enoxolone, Fluazacort, Flucloronide, Flumethasone, Flunisolide, Fluocinolone Acetonide, Fludrocortisone, Fluocinonide, Fluocortin Butyl, Fluocortolone, Fluorometholone, Fluperolone, Fluprednidine, Fluprednisolone, Flurandrenolide, Formocortal, Halcinonide, Halomethasone, Halopredone, Hydrocortamate, Hydrocortisone, Limethasone, Mazipredone, Medrysone, Meprednisone, Methyolprednisolone, Methyolprednisolone hemisuccinate, Mometasone Furoate, Paramethasone, Prednicarbate, Prednisolone, Prednisolone palmitate, Prednisolone phosphate, Prednisone, Prednival, Prednylidene, Tixocortal, and Triamcinolone.

An embodiment of the invention is a liposome, wherein said at least one active ingredient is an immuno suppressive compound comprising a steroid selected from the group consisting of Betamethasone hemisuccinate, Dexamethasone palmitate, Dexamethasone phosphate, Limethasone, Methyolprednisolone hemisuccinate, Prednisolone palmitate, and Prednisolone phosphate.

An embodiment of the invention is a liposome, wherein said at least one active ingredient is an immuno-suppressive compound comprising a small molecule inhibitor acting on intracellular targets selected from the group consisting of c-Fms, PDGFRα, Abl, PDGFRβ, NFκB, IκB, JAK1, JAK2, JAK3, GSK3, p38 MAPK, JNK, KIT, EGFR, ERBB2, ERBB4, VEGFR1, VEGFR2, VEGFR3, FLT3, PKCβ, RAF1, CDK1, CDK2, CDK4, NLRP3, IRF3, STAT1, STAT2, STAT3, STAT4, STAT5, STAT6, Hsp90, Hsp70, PI3K, mTOR, AKT, DNA-PK, ATM, AMPK, PDK-1, S6 kinase, RIP2, TRIF, MYD88, TAK1.

An embodiment of the invention is a liposome, wherein said at least one active ingredient is an immuno-suppressive compound comprising a small molecule inhibitor acting on intracellular targets selected from the group consisting of indomethacin, CLI-095 (C15H17ClFNO4S, CAS#243984-11-4), Bay11-7082 (C10H9NO2S, CAS#19542-67-7), Triptolide (PG 490), CGP53716, SU9518, PD166326, Celastrol, Tripterin, BIRB-796 (Doramapimod), SB 203580, SB202190, VX-702, NVP-BEZ235, GDC-0980 (RG7422), Ridaforolimus (Deforolimus, AP23573), Nilotinib (Tasigna,AMN107), Sorafenib tosylate (Nexavar), Dasatinib (Sprycel, BMS-354825), MLN518 (CT53518), Vatalanib (PTK787 / ZK222584), OSI-930, AZD2171, Pazopanib (Votrient), IPI-504 (retaspimycin), Deforolimus (Ridaforolimus), GDC-0980 (RG7422, C₂₃H₃₀N₈O₃S, CAS#1032754-93-0), Palomid 529 (C₂₄H₂₂O₆ CAS#914913-88-5), Imatinib mesylate (Gleevec/Glivec), Everolimus (Afinitor, RAD001), Sirolimus (Rapamune, rapamycin), Temsirolimus (Torisel, CCI-779), Bortezomib (Velcade), Gefitinib (Iressa), Canertinib (CI-1033, CAS# 267243-28-7, C₂₄H₂₅ClFN₅O₃), Erlotinib hydrochloride (Tarceva), Pelitinib (EKB-569) (C₂₄H₂₃ClFN₅O₂, CAS#257933-82-7), Vandetanib (Zactima, ZD6474, C₂₂H₂₄BrFN₄O₂, CAS No.: 443913-73-3), Sunitinib (Sutent, SU-11248, C₂₂H₂₇FN₄O₂.C₄H₆O₅, CAS No.: 341031-54-7), Tandutinib (MLN518), C₃₁H₄₂N₆O₄, CAS No.: 387867-13-2, Roscovitine (Seliciclib), C₁₉H₂₆N₆O, CAS No.: 186692-46-6.

An embodiment of the invention is a liposome, wherein said at least one active ingredient is an anti-infectious compound selected from the group consisting of Rifampicin, dideoxycytidine-5'-triphosphate, Clarithromycin, acyclovir, ciprofloxacin, fusidin, gentamicin, chloramphenicol, levofloxacin, oxytetracyclin, tobramycin, natriumcromoglicat, Amoxicillin, Ampicillin., Pivampicillin, Ertapenem, Meropenem, Doripenem, Cefotaxim, Ceftazidim, Ciprofloxacin, Valaciclovir, Efavirenz, Emtricitabin, Tenofovirdisoproxil, Rilpivirine, Penicillin, Trimethoprim-sulfamethoxazole, Rifampicin, Etambutol, Isoniazid, Pyrazinamide, Voriconazole, Amphotericin B, Caspofungin, Flucytosine, Itraconazole, Doxycyclin, Sulfonamides, and Sulfamethoxazole.

An embodiment of the invention is a liposome, further comprising at least one antigen as active ingredient.

An embodiment of the invention is a liposome, wherein said at least one antigen is selected from the group consisting of a cancer antigen, a self- or autoimmune antigen, a microbial antigen, an allergen, or an environmental antigen.

For monocyte specific targeting of drugs, positively liposomes will likely be phagocytosed by the monocyte, and the drug entrapped inside the liposomes will be released inside the monocyte, allowing the drug to exert its intracellular function. For immune stimulating compounds like e.g. agonists towards intracellular receptors like pattern recognition receptors (PRRs), these molecules will be released once inside the cell, and activate the relevant receptor, which may result in immune stimulatory monocytes.

### TEST OF LIPOSOMES FOR MONOCYTE TARGETING:

The preferred liposomes for specific monocyte targeting show a zeta potential between 0-60 mV, 20-50 mV, 23-45 mV, 31-41 mV, 32-38 mV when measured on a ZetaPALS zeta potential analyzer (Brookhaven Instruments Coorporation, Holtsville, NY) in a buffer consisting of 300 mM glucose, 10 mM HEPES, 1 mM CaCl₂ in MilliQ water, pH 7.4. In addition, the preferred liposome for specific monocyte targeting is tested using the following test system:
Fresh whole human blood is drawn from healthy donors using BD vacutainers (Cat# 366450) with EDTA as anticoagulant. A volume of 10 µL liposome (5 mM) is added to a volume of 190 µL (final 250 µM) fresh blood in 1.5 mL eppendorf tubes and incubated at 37C with gentle rotation for 60 minutes. Red blood cells are lysed in 4 mL BD Pharm lysis buffer in the dark at room temperature for 15 minutes in BD serum Vacutainers (Cat#367614), centrifuged at 200g for 5 min and resuspended in 1 mL Pharm lysis buffer. After 5 min incubation in the dark at RT, samples are centrifuged at 200 g for 5 min, resuspended in 1 mL FACS buffer (PBS supplemented with 1% BSA), immediately centrifuged at 200 g for 5 min, resuspended in 400 µL FACS buffer and transferred to a 96 well plate with round bottom (Nunc, Roskilde, DK). Unspecific binding is blocked by human IgG for 10 min on ice before the cells are incubated in 100 µL with primary pre-conjugated antibody or isotype control for 60 minutes on ice, centrifuged at 500 g for 5 min at 4°C, and washed twice in 200 µL FACS buffer. Cells are resuspended in 150 µL FACS buffer, before being subjected to flow cytometric analysis, which can be carried out on a BD FACSArray bioanalyzer. The cellular uptake of liposomes is determined based on the fluorescence of DOPE-rhodamine B (RhB) incorporated into the liposomal membrane. The total amount of liposome associated with cells (indicated as 'uptake' and include cell membrane bound liposomes and liposomes already internalized) is estimated using excitation at 532 nm and emission at 564-606 nm. Liposomal uptake in different cell populations in peripheral blood is analyzed based on the following markers: CD14 (monocytic marker), CD15 (granulocytic marker), CD3 (T-lymphocytic marker), CD19 (B-lymphocytic marker), and CD56 (natural killer cell marker). Data can be analyzed by BD FACSDiva Software v5.0.2. Unstained cells are used as negative controls, and cells with fluorescence higher than the control cells were considered positive. The same gate was used to analyze all cell populations in each experiment.

### Antigens

The antigen may be without limitation a cancer antigen, a self or autoimmune antigen, a microbial antigen, an allergen, or an environmental antigen. The antigen may by peptide, lipid, or carbohydrate in nature, but it is no so limited.

Cancer Antigens: A cancer antigen is an antigen that is expressed preferentially by cancer cells (i.e., it is expressed at higher levels in cancer cells than on non-cancer cells) and in som instances it is expressed solely by cancer cells. The cancer antigen may be expressed within a cancer cell or on the surface of the cancer cell. The cancer antigen may be MART- 1/Melan-A, gp100, adenosine deaminase-binding protein (ADAbp), FAP, cyclophilin b, colorectal associated antigen (CRC) 0017-1A/GA733, carcinoembryonic antigen (CEA), CAP-1, CAP-2, etv6, AML 1, prostate specific antigen (PSA), PSA-1, PSA-2, PSA-3, prostate-specific membrange antigen (PSMA), T cell receptor/CD3-zeta chain, and MAGE-A1, MAGE-A2, MAGE-A3, MAGE-A4, MAGE-A5, MAGE-A6, MAGE-A7, MAGE-A8, MAGE-A9, MAGE-A10, MAGE-A11, MAGE-A12, MAGE-Xp2, (MAGE-B2), MAGE-Xp3 (MAGE-B3), MAGE-Xp4 (MAGE-B4), MAGE-C1, MAGE-C2, MAGE-C3, MAGE-C4, MAGE-C5, GAGE-1, GAGE-2, GAGE-3, GAGE-4, GAGE-5, GAGE-6, GAGE-7, GAGE-8, GAGE-9. BAGE, RAGE, LAGE-1, NAG, GnT-V, MUM-1, CDK4, tyrosinase, p53, MUC family, HER2/neu, p21ras, RCAS1, a-fetoprotein, E-cadherin, a-catenin, β-catenin, y-catenin, pl120ctn, gp100, PRAME, NY-ESO-1, cdc27, adenomatous polyposis coli protein (APC), fodrin, Connexin 37, Ig-idiotype, p15, gp75, GM2 ganglioside, GD2 ganglioside, human papilloma virus proteins, Smad family of tumor antigens, Imp-1, P1A, EBV-encoded nuclear antigen (EBNA)-1, brain glycogen phosporylase, SSX-1, SSA-2 (HOM-MEL-40), SSX-1, SSX-4, SSX-5, SCP-1 and CT-7, CD20, and c-erbB-2. (Amll:cyclophilin b), B cell lymphoma (Ig-idiotype); Burkitt's (Non-Hodgkin's) lymphoma (CD20): glioma (E-cadherin:α-catenin, β-catenin, γ-catenin, p120ctn), bladder cancer (p21ras), biliary cancer (p21ras), breast cancer (MUC family; HER2/neu; e-erbB2), cervical carcinoma (p53; p21ras), colon carcinoma (p21ras; HER2/neu; c-erbB-2; MUC family), gastric cancer (HER2/neu; c-erbB-2; ga733 glycoprotein), hepatocellular cancer, melanoma (p15 protein, gp75, oncofetal antigen , GM2 and GD2 gangliosides), myeloma (MUC family, p21ras), non-small cell lung carcinoma (HER2/neu;c-erbB-2), nasopharyngeal cancer (Imp-1; EBNA-1), ovarian cancer (MUS family;Her2/neu;c-erbB-2), squamous cell cancers of cervix and esophagus (viral products such as human papilloma virus proteins and non-infectious particles), testicular cancer (NY-ESO-1), T cell leukemia (HTLV-1 epitopes).

Microbial Antigens: Microbial antigens are antigens derived from microbial species such as without limitation bacterial, viral fungal, parasitic and mycobacterial species. As such, microbial antigens include bacterial antigens, viral antigens, fungal antigens, parasitic antigens, and mycobacterial antigens. Examples of bacterial, viral, fungal, parasitic and mycobacterial species are provided herein. The microbial antigen may be part of a microbial species or it may be the entire microbe. In one embodiment, the bacterial antigen is derived from a bacterial species selected from the consisting of *E. coli, Staphylococcal, Chlamydia, Streptococcal, Pseudomonas, Clostridium difficile, Legionella, Pnetanococcus. Haemophilus, Klebsiella, Enterobacter, Citrobacter, Neisseria, Meningococcus B, Shigella, Salmonella, Listeria, Pasteurella, Streptobacillus, Spirillum, Treponema, Actinomyces, Borrelia, Corynebacterium, Tuberculosis, Norcardia, Gardnerella, Campylobacter, Spirochaeta, Proteus, Bacteriodes, Yersenia pestis, H. pylori,* and *anthrax.*

In another embodiment, the viral antigen is derived from a viral species selected from the gruop consisting og *HIV, Coronavirus, Herpes simples virus 1, Herpes simplex virus 2, cytomegalovirus, Dengue virus, Ebola virus, hepatitis A virus, hepatitis B virus, hepatitis C virus, hepatitis E virus, human papilloma virus, human Metapneumoniavirus, Epstein Barr virus, rotavirus, adenovirus, influenza virus (universal, H1N1v, H7N1, H9N2), Pneumococcus, Para* influenza *virus, respiratory syncytial virus (RSV), varicella-zoster virus, small pox, monkey pox, West Nile virus* and *SARS.*

In yet another embodiment, the fungal antigen is derived from a fungal species that causes an infection selected from the group consisting of *candidiasis, ringworm, histoplasmosis, blastomycosis, paracoccidioidomycosis, cryptococcosis, aspergillosis, chromomycosis, mycetoma infections, pseudallescheriasis, and tinea versicolor* infection.

In still another embodiment, the parasitic antigen is derived from a parasite species selected from the group consisting of amebiasis, *trypanosome cruzi, Fasciolia, Leishmania, Plasmodium. Onchocercia, Paragonimia, Trypanosoma brucei, Pneumocystis, Trichomonas vaginalis, Taenia, Hymenolepsis, Echinococcus, Schistosoma, neurocysticercosis, Necator americanus,* and *Trichuris trichuria.*

The mycobacterial antigen may be derived from a mycobacterial species such as *M*. *tuberculosis* and *M. leprae,* but not so limited.

Allergen: An allergen is an agent that can induce an allergic or asthmatic response in a subject. Allergens include without limitation pollens, insect venoms, animal dander dust, fungal spores and drugs (e.g. penicillin). Examples of natural, animal and plant allergens include but are not limited to proteins specific to the following genera: canine *(Canis familiaris); Dermatophagoides (e.g.Dermatophagoides farina); Felis (Felis domesticus); Ambrosia (Ambrosia artemi isfolia; Lolium (e.g. Lolium perenne or Lolium multiflorum); Cryptomeria (Cryptomeria japonica): Alternaria (Alternaria alternate); Alder, Alnus (Alnus gultinoasa); Betula (Betula verrucosa); Quercus (Quercus alba); Olea (Olea Europa); Artemisia (Artemisia vulgaris): Plantago* (e.g. *Plantago lanceolata); Parietaria* (e.g. *Parietaria officinalis of Parietaria judaica); Blattella* (e.g. *Blattella germanica); Apis* (e.g. *Apis multiflorum); Cupressus* (e.g. *Cupressus sempervires, Cupressus arizonica and Cupressus macrocarpa); Juniperus* (e.g. *Juniperus sabinoides, Juniperus virginiana, Juniperus communis* and *Juniperus ashei); Thuya* (e.g. *Thuya orientalis); Chamaecyparis* (e.g. *Chamaecyparis obtuse); Periplaneta* (e.g. *Periplaneta Americana); Agropyron* (e.g. *Agropyron repens); Secale* (e.g. *Secale cereal); Triticum* (e.g. *Triticum aestivum); Dactylis* (e.g *Dactylis glomerata); Festuca* (e.g. *Festuca elatior); Poa* (e.g. *Poa pratensis of Poa compressa); Avena* (e.g. *Avena sativa); Holcus* (e.g. *Holcus lanatus); Anthoxanthum* (e.g. *Avena sativa); Holcus* (e.g. *Holcus lanatus); Anthoxanthum* (e.g. *Anthoxanthum odoratum); Arrhenatherum* (e.g. *Arrhenatherum elatius); Agrostis* (e.g. *Agrostis alba); Phleum* (e.g. *Phleum pretense); Phalaris* (e.g. *Phalaris arundinacea); Paspalum* (e.g. *Paspalum notatum); Sorghum* (e.g. *Sorghum halepensis);* and *Bomus* (e.g. *Bromus inermis)*

For example, an antigen involved in any one or more of the following autoimmune diseases or disorders can be used in the present invention; diabetes, diabetes mellitus, arthritis (including rheumatoid arthritis, juvenile rheumatoid artritis, osteoarthritis, psoriatic arthritis), multiple sclerosis, myasthenia gravis, systemic lupus erythematosis, autoimmune thyroiditis, dermatitis (including atopic dermatitis and eczematous dermatitis), psoriasis, Sjogren's Syndrome, including keratoconjunctivitis sicca secondary to Sjogren's Syndrome, alopecia areata, allergic responses due to arthropod bite reactions, Crohn's disease, aphthous ulcer, iritis, conjunctivitis, keratoconjunctivitis, ulcerative colitis, asthma, allergic asthma, cutaneous lupus erythematosus, scleroderma, vaginitis, proctitis, drug eruptions, leprosy reversal reactions, erythema nodosum leprosum, autoimmune uveitis, allergic encephalomyelitis, allergic rhinitis, acute necrotizing hemorrhagic encephalopathy, idiopathic bilateral progressive sensorineural hearing loss, aplastic anemia, pure red cellanemia, idiopathic thrombocytopenia, polychondritis, Wegener's granulomatosis, chronic active pepatitis, Stevens Johnson syndrome, idiopathic sprue, lichen planus, Crohn's disease, Graves ophtalmopathy, sarcoidosis, primary biliary cirrhosis, uveitis posterior, and interstitial lung fibrosis. Examples of antigens involved in autoimmune disease include glutamic acid decarboxylase 65 (GAD 65), nativeDNA, myelin basic protein, myelin proteolipid protein, acetylcholine receptor components, thyroglobulin, and thyroid stimulating hormone (TSH) receptor.

Other conditions equally envisioned in the present invention are characterised as systemic inflammation and/or infections and can be associated with trauma, burns, surgery, coronary artery disease, coronary atherosclerosis, atherosclerosis, Infarct Inflammation and Repair after Myocardial Infarction, cardiac ischemia-reperfusion injury, myocardial inflammation, myocardial ischemia, cardiovascular disease, brain infarct, hemorrhagic shock, reperfusion lesion, dialysis, shock and/or bacterial or viral infection, extracorporeal membrane oxygenation (ECMO), Shock, systemic inflammatory response syndrome (SIRS), sepsis, multi organ failure, rejection of an implant such as organ and/or prostheses, heart and lung bypass surgery,chronic inflammation, asthma and/or stress related disease in the lung, bacterial infection, gangrene, soft tissue infection, and wound infection.

### Pharmaceutical compositions of the invention

As discussed above, the liposomes of the present invention are useful as constituents of a pharmaceutical formulation of the invention. Any form of such formulation which is suitable for intravenous administration to a mammal is contemplated.

The pharmaceutical formulation according to the invention is preferably in the form of a solution, dispersion, suspension, lyophilisate, or frozen form.

### Therapeutic uses or methods

Also part of the invention is the liposome or the lipid-based drug delivery system or the pharmaceutical composition of the invention for use as a pharmaceutical. In particular, these aspects of the invention may be for use in prophylactic or therapeutic treatment or amelioration of a monocytic associated condition, such as:
Sepsis:
   Septic shock is the most common cause of death in intensive care units, and the mortality rate ranges between 25-50 % (Kumar, V, et al., (2007). Robbins Basic Pathology (8th ed.). Saunders Elsevier. pp. 102-103). Activation of monocytes plays an important role for the survival of patients, where monocytes are the major source of secreted cytokines critical for disease development, involving mainly IL1β, IL-6 and TNFa. The survival or septic patients correlates with the level of IL-6 seen in serum from these patients for the first 48 h after onset of sepsis. For patients with high IL-6 measured within the first 48 h after diagnosis (>1000 pg/mL), the mortality rate is 50 %, whereas low IL-6 (<1000 pg/mL) correlates with good survival (100 % survival at day 28) (Spittler A., et al., Clinical Infections Diseases, 2000, 31;1338-42). Administration of low dose glucocorticoids reduced the mortality rate in patients with sepsis (Djillali A., et al. JAMA 2002, 288, no7, 862-872), indicating that glucocorticoid treatment, and in particular targeted liposomal glucocorticoid treatment as disclosed in this present application, targeted to one of the major effector cells in sepsis; the monocytes, may be a promising therapeutic opportunity, in particular when administered to patients with high IL-6 levels (>1000 pg/mL) the first 48 h after diagnosis and/or onset.
Kawasaki Disease:
   Kawasaki Disease (KD) is an autoimmune inflammation of the mid-sized arteries. It affects many organ systems, mainly those including the blood vessels, skin, mucous membranes and lymph nodes; most serious effect is on the heart where it can cause fatal coronary artery aneurysms in untreated children. KD is mainly a pediatric disease, which is likely induced by an earlier infection. The inflammation spreads completely around the artery; as a result, structural components of the artery undergo intense damage; the artery then begins to dilate. Inflammatory cell infiltration continues until about the 25th day of the disease, after which the inflammatory cells gradually decrease in number. KD arteritis is characterized by granulomatous inflammation that consists of severe accumulation of monocytes/macrophages (Matsubara T., et al., Clin Exp Immunol, 141:381-387). Aberrant activation of monocytes/macrophages is thought to be involved in the formation of vascular lesions. KD progress up to 3-4 weeks, and involves monocyte/macrophage activation with increased TNFa, IL-2R, IL-1, IL-6 and NO-synthase induced.( Furukawa S, et al., Nihon Rinsho. 2008 Feb;66(2):258-64).

### Coronary arteritis

Coronary arteritis begins 6-8 days after the onset of KD (Takahashi K, Clin Exp Immunol. 2011 May;164 Suppl 1:20-2). Intravenous immunoglobulin (IVIG) is the standard treatment for Kawasaki disease, often supplemented with aspirin which is administered for up to two months. Corticosteroids have also been used with success, and is comparable to or slightly more potent than IVIG in reduction of cytokine levels, especially when other treatments fail or symptoms recur, but may cause other side effects. (Miura M et al., Eur J Pediatr. 2008 Oct;167(10):1119-23). Thus, liposomal targeting to monocytes in KD with immune suppressing liposomes may improve the immuno suppressive effect and disease progression, at the same time reducing the systemic side effects seen for administration of free corticosteroids.

### Sarcoidosis

Sarcoidosis is a disease of unknown cause, in which abnormal collections of chronic inflammatory cells (granulomas) form as nodules in multiple organs. Between 30 and 70% of patients do not require therapy. ^{]}Corticosteroids, most commonly prednisolone, have been the standard treatment for many years. In some patients, this treatment can slow or reverse the course of the disease, but other patients do not respond to steroid therapy. About 10% develop serious disability. Lung scarring or infection may lead to respiratory failure and death (Chen ES. Et al,. Nat Rev Rheumatol. 2011 Jul 12;7(8):457-67).Increased TLR expression and enhanced secretion of pro-inflammatory cytokines in blood mononuclear cells from sarcoidosis patients after combined TLR2 and NOD2 stimulation may be related to the pathogenesis of sarcoidosis. Thus, cationic liposomes coformulated with immunosuppressive compounds like corticosteroids targeting monocytes in sarcoidosis patients, may improve disease development in severe cases of sarcoidosis. (Wikén M., et al,. J Clin Immunol. 2009 Jan;29(1):78-89)

Other conditions equally envisioned in the present invention are characterized as systemic inflammations and/or infections and can be associated with trauma, burns, surgery, Dialysis, shock and/or bacterial or viral infection. In a preferred embodiment of the invention, the systemic condition is associated with a disease selected from the group consisting of extracorporeal membrane oxygenation (ECMO), shock, coronary artery disease, coronary atherosclerosis, atherosclerosis, Infarct Inflammation and Repair after Myocardial Infarction, cardiac ischemia-reperfusion injury, myocardial inflammation, myocardial ischemia, cardiovascular disease, brain infarct, hemorrhagic shock, reperfusion lesion, systemic inflammatiory response syndrome (SIRS), sepsis, multi organ failure, rejection of an implant such as an organ and/or prostheses, heart and lung bypass surgery, chronic inflammation, asthma and /or stress related diseases in the lung, bacterial infection, gangrene, soft tissue infection and wound infection, systemic inflammatory response syndrome, SLE, Hemolytic uremic syndrome, Hemophagocytic lymphohistiocytosis, Alveolar proteinosis, Systemic sclerosis, Osteomyelitis, Organ transplant rejection (GVHD), Lupus erythematosus, Sjögrnes syndrome, Glomerulonephritis, Hemolytic uremic syndrome, erysipelas, Hemophagocytic lymphohistiocytosis

In addition, the aspects of the invention may be for use in prophylactic or therapeutic treatment or amelioration of a local and/or chronic inflammation, such as inflammations of:
Myocardial infarction associated with ischemia reperfusion injury:
   Myocardial infarction (MI) may induce transient ischemia, causing necrosis in the heart tissue. The necrotic tissue must be removed within the first days after the MI, which occurs when NK cells, macrophages and monocytes migrate into the tissue and produce proteolytic enzymes and inflammatory cytokines and chemokines including IL-8, MCP-1 and CCL5 and reactive oxygen species (ROS). The first days after MI, the damaged tissue become infiltrated by inflammatory monocytes required for phagocytosis of necrotic tissue, trigger angiogenesis and initiate collagen synthesis, factors important for the later tissue healing of the infarct area. However, if inflammatory monocytes persist in the healing tissue, reparative monocytes will not be able to invade and restore the tissue. Therefore, the balance between inflammatory and reparative monocytes is important for a successful healing process after MI (Nahrendorf M, et al., Circulation, 2010, 121:2437-2445). Thus, patients who suffer from MI with high levels of inflammatory monocytes in the first course after MI, will benefit from treatment with monocyte targeting liposomes with immunosuppressive compounds like glucocorticoids, which will suppress production of proinflammatory cytokines, chemokines and reactive oxygen species at the site of injury, thereby reducing the myocardial inflammation and damaging effect from inflammatory monocytes.
Atherosclerosis:
   Atherosclerosis is an arterial disease which involves inflammation of the arterial wall, and deposition of cholesterol, lipids and inflammatory macrophages, forming a plaque. The plaques may rupture, leading to thrombosis, myocardial infarction and stroke. Monocytes accumulate at sites of inflammation and have been reported to enter atherosclerotic plaques, where they likely differentiate to form cells and macrophages (Wollard KJ,. Geissmann F., Nar Rev Cardiol., 2010;7(2):77). In addition, the monocyte derived cytokines IL-6 and TNFα, are elevated in atherosclerotic patients (Correa CR., et al. Journal of inflammation, 2011,8:23), and therapies to inhibit monocyte recruitment to plaques has been suggested as a viable strategy to reduce plaque formation (Potteaux S. et al., Journal of clinical investigation, 2011;5;121, 2025-2036). Monocytes treated with glucocorticoids show reduced migration and adhesion to inflamed endothelial cells, indicating that monocyte targeting liposomes with coformulated glucocorticoids may be a good strategy to reduce or even resolve atheroschlerotic plaques.

### Rheumatoid arthritis

Rheumatoid arthritis (RA) is an autoimmune disease with inflammation and deformation of the joints. Monocytes, macrophages, T and B-cells accumulate in the synovium where they play an important role for joint destruction. The involvement of inflammatory monocytes for RA development is underlined by the fact that monocytes secrete large amounts of TNFα, and blocking this cytokine is one of the most efficient treatments of RA, second, monocyte depletion have shown beneficial effect for RA patients (Sanmarti R. et asl., Rheumatology. 2005 Sep;44(9):1140-4), and third, blocking monocyte migration by neutralizing migration markers CCR1,2,3,5 and 9 has been suggested a promising strategy for alleviation of RA symptoms (Schmutz C. et al., Arthritis Research & Therapy, 2010, 12: R161). Thus, liposomes able to deliver immunosuppressive compounds like glucocorticoids may be a promising strategy in order to 1) reduce monocyte migration to the inflamed joint through downregulation of migration receptors, 2) reduce production of inflammatory cytokines like e.g. TNFα.

### Osteoarthritis

Osteoarthritis (OA) is the most common joint disorder worldwide, and it has an enormous socioeconomic impact. Although osteoarthritis (OA) involves joint inflammation, the inflammation in OA is less severe than in RA. However, mononuclear cells from OA joints produce inflammatory cytokines like IL-1 and TNFα which plays a major role for OA progression (Femandes J.C. et al., Biorheology. 2002;39(1-2):237-46). Neutralisation of IL-1 and/or TNFα seems to reduce disease symptoms, at least in experimental animal models (Calich AL, Clin Rheumatol. 2010 May;29(5):451-5). Thus, liposomes able to deliver immunosuppressive compounds like glucocorticoids may be a promising strategy in order to 1) reduce monocyte migration to the inflamed OA joint through downregulation of migration receptors, 2) reduce production of inflammatory cytokines like IL-1 and TNFα.

In addition, the aspects of the invention may be for use in prophylactic or therapeutic treatment or amelioration of the following conditions or diseases:Crohns Disease, atopic eczema, Pulmonary fibrosis, Inflammations of the skin, Alveolar proteinosis, COPD, diabetes, diabetes mellitus, arthritis (including rheumatoid arthritis, juvenile rheumatoid artritis, osteoarthritis, psoriatic arthritis), multiple sclerosis, myasthenia gravis, systemic lupus erythematosis, autoimmune thyroiditis, dermatitis (including atopic dermatitis and eczematous dermatitis), psoriasis, Sjogren's Syndrome, including keratoconjunctivitis sicca secondary to Sjogren's Syndrome, alopecia areata, allergic responses due to arthropod bite reactions, aphthous ulcer, iritis, conjunctivitis, keratoconjunctivitis, ulcerative colitis, asthma, allergic asthma, cutaneous lupus erythematosus, scleroderma, vaginitis, proctitis, drug eruptions, leprosy reversal reactions, erythema nodosum leprosum, autoimmune uveitis, allergic encephalomyelitis, acute necrotizing hemorrhagic encephalopathy, idiopathic bilateral progressive sensorineural hearing loss, aplastic anemia, pure red cellanemia, idiopathic thrombocytopenia, polychondritis, Wegener's granulomatosis, chronic active pepatitis, Stevens Johnson syndrome, idiopathic sprue, lichen planus, Graves ophtalmopathy, sarcoidosis, primary biliary cirrhosis, uveitis posterior, and interstitial lung fibrosis.

In addition, the aspects of the invention may be for use in prophylactic or therapeutic treatment or amelioration of a condition where immune stimulation is preferred, such as a condition of cancer, infection or disease which requires administration of a vaccine or immune stimulation, such as (cancer solid tumors, vaccines against cancer and infectious disease).

### Tolerogenic vaccines

In addition, the aspects of the invention may be for use in tolerogenic vaccines in which an antigen is encapsulated or associated with the monocyte targeting liposome, either by passive or active loading or by anchoring in the liposomal membrane. In addition, a tolerogenic liposome may include one or more tolerance inducing compounds selected from the group consisting of vitamin D3 (1,25-dihydroxyvitamin D₃) and retinoic acid (all-trans and 9-cis retinoic acid), Betamethasone hemisuccinate, Dexamethasone palmitate, Dexamethasone phosphate, Limethasone, Methylprednisolone hemisuccinate, Prednisolone palmitate, and Prednisolone phosphate and their related synthetic or natural analogues. Such formulation administered intravenously may induce an antigen specific torlerance inducing monocyte, that may differentiate into af tolerance inducing macrophage or dendritic cell. Tolerance inducing monocyte targeting liposomes may be used for treatment or alleviation of immune disorders involving a self-antigen (autoimmune diseases) or a foreign antigen (Allergies), or in hypersensitivity reactions. Examples of such dieseases includes autoimmune conditions like e.g Crohns Disease, atopic eczema, diabetes, arthritis, multiple sclerosis, myasthenia gravis, systemic lupus erythematosis, psoriasis. Tolerogenic vaccines for treatment of allergies may include asthma, allergic cough, allergic rhinitis and conjunctivitis, atopic eczema, dermatitis, drug allergies, urticaria, hives, insect bite allergy, dietary allergy, such as fish, milk, wheat, peanut, apple, nut allergy.

All cited references are incorporated in their entirety herein.

### EXAMPLE 1

### Liposome preparation

Unilamellar fully hydrated liposomes were made from mixtures of 1-palmitoyl-2-oleoyl-sn-glycero-3-phosphocholine (POPC), 1-palmitoyl-2-oleoyl-sn-glycero-3-phosphoglycerol (POPG), 1,2-dioleoyl-3-trimethylammonium-propane (DOTAP) and 1,2-dioleoyl-sn-glycero-3-phosphoethanolamine-N-[methoxy(polyethylene glycol)-2000] (DOPE-PEG2000). As a fluorescence marker to measure presence of liposomes in biological systems, 0.5% 1,2-dioleoyl-sn-glycero-3-phosphoethanolamine-N-rhodamine (DOPE-RhB) was mixed with the lipids as a tracer. The molar ratios of each lipid in the liposomes are outlined in Fig. 1A. All lipids were all obtained from Avanti Polar lipids. Briefly, appropriate weighed amounts of POPC, POPG, DOTAP and DOPE-PEG2000 were dissolved in chloroform. The solvent was removed by a gentle stream of N₂ and the lipid films were dried overnight under low pressure to remove trace amounts of solvent. Multilamellar vesicles were prepared by dispersing the dried lipids in a buffer solution containing: 150 mM KCL, 10 mM HEPES (pH = 7.5), 1 mM NaN₃, 30 µM CaCl₂ and 10 µM EDTA. The multilamellar vesicles were extruded ten times through two stacked 100 nm pore size polycarbonate filters as described by Mayer et al., Biochim. Biophys. Acta, 858, 161-168.

### EXAMPLE 2

### Characterization of liposome size and surface charge dependent on composition

Liposomes prepared as outlined in Fig. 1A were prepared with the attempt to design liposomes with ability to be recognized and taken up by monocytes but not other cells in the blood. We designed liposomes with 0-50% net positive charge (formulation 6-12 in figure 1A), together with control liposomes with negative or nearly neutral charge (formulation 1-5 in figure 1A). The liposomes were prepared as described in example 1, and their size measured in nanometer (nm) by dynamic light scattering on a ZetaPALS zeta potential analyzer from Brookhaven Instruments in a buffer consisting of 300 mM glucose, 10 mM HEPES, 1 mM CaCl₂ in MilliQ water, pH 7.4. The liposomes showed sizes between 110 ± 20 nm in diameter (Figure 1B). The surface charge (Zeta potential) of the liposomes was measured in mV and showed surface charge dependent on lipid composition (figure 1C). Addition of the negatively charged POPG (10 molar percent) in the liposome, showed negative surface charge of -13 mV (figure 1C, composition 1). High content of the neutral lipid POPC (100 molar percent) in the liposome showed neutral charge (figure 1C, composition 3). Increased amounts of DOTAP to 10% (molar percent 90: 10), showed a net positive charge of nearly 38 mV (figure 1B, composition 8), whereas the highest amount of DOTAP at 50 mol% (molar percent 50:50), showed a net positive charge of 52 mV (figure 1C, composition 12).

### EXAMPLE 3

### Liposome targeting to monocytes dependent on liposome composition

The cellular uptake of modified POPC liposome formulations was determined based on fluorescence of RhB incorporated into the liposomal membrane. The total amount of liposome associated with cells (indicated as 'uptake' and include cell membrane bound liposomes and liposomes already internalized) was estimated using excitation at 532 nm and emission at 564-606 nm. Liposomal uptake in five different cell populations in whole blood was analyzed. The following markers were used to distinguish the different populations: CD14 (monocytic marker), CD15 (granulocytic marker), CD3 (T-lymphocytic marker), CD19 (B-lymphocytic marker), and CD56 (natural killer cell marker). Whole blood was obtained from healthy volunteers by standard methods in BD Vacutainer containing ethylenediaminetetraacetic acid (EDTA, 366450). Briefly, 10 µl liposome preparations (formulation 1-5 and 8 in fig. 1A) were added to 200 µl fresh whole blood in 1.5 ml eppendorf tubes. Samples were incubated for 1 h at 37°C with rotation. Red blood cells (RBC) were lysed in 4 ml BD Pharm lysis buffer in the dark at room temperature (RT) for 15 min in BD serum Vacutainer (367614), centrifuged at 200 g for 5 min, and resuspended in 1 ml Pharm lysis buffer. After 5 min incubation in the dark at RT, samples were centrifuged at 200 g for 5 min, resuspended in 1 ml FACS buffer (PBS supplemented with 1% BSA), immediately centrifuged at 200 g for 5 min, resuspended in 400 µl FACS buffer and transferred to a 96 well plate with round bottom (Nunc). Unspecific binding were blocked by human IgG for 10 min on ice before the cells were incubated in 100 µl with primary pre-conjugated antibody or isotype control (Table 1) for 60 minutes on ice, centrifuged at 600 g for 5 min at 4°C, and washed twice in 200 µl FACS buffer. Cells were resuspended in 150 µl FACS buffer, before being subjected to flow cytometric analysis, which were carried out on a BD FACSArray bioanalyzer. Data was analyzed by BD FACSDiva Software v5.0.2. Unstained cells were used as negative control, and cells with fluorescence higher than the control cells were considered positive. The same gate was used to analyze all cell populations. Negatively charged and neutral liposomes were not taken up by cells (fig. 2). Anionic and neutral liposomes were not taken up by any of the cell populations. The cationic formulation containing DOTAP were taken up in a significantly higher amounts of CD14 positive monocytes (p<0.0001) than in the other cell populations. The addition of 10 mol% PEG inhibited some of the uptake of cationic liposomes in CD14 positive monocytes. Based on these results, only two types of formulations (POPC and POPC: DOTAP) will be used in future experiments.

### EXAMPLE 4

### Liposome targeting to monocytes dependent on liposome incubation time

Whole blood was obtained from healthy volunteers as described in example 3. Briefly, 10 µl liposome preparations (formulation 3 and 8 in fig. 1A) were added to 200 µl fresh whole blood in 1.5 ml eppendorf tubes. Samples were incubated for 5 min, 15 min and 1 h at 37°C with rotation. Red blood cells (RBC) were lysed and cells were prepared for FACS analysis as described in example 3. Data was analyzed by BD FACSDiva Software v5.0.2. Unstained cells were used as negative control, and cells with fluorescence higher than the control cells were considered positive. The same gate was used to analyze all cell populations (Fig 3). Time dependent increase in cellular uptake was observed in monocytes when the liposomes contained 10% DOTAP (Fig. 3B). The uptake in monocytes was significantly higher than the uptake in the other four cell populations at all 3 time points (p<0.0001).

### EXAMPLE 5

### Liposome targeting to monocytes dependent on liposome charge properties

The effect of positive charge on the cellular uptake was tested using liposome formulations containing 0-50 mol% DOTAP (Fig. 5A). Whole blood was obtained from healthy volunteers as described in example 3. Briefly, 10 µl liposome preparations (formulation 3 and 6-12 in fig. 1A) were added to 200 µl fresh whole blood in 1.5 ml eppendorf tubes. Samples were incubated for 1 h at 37°C with rotation. Red blood cells (RBC) were lysed and cells were prepared for FACS analysis as described in example 3. Data was analyzed by BD FACSDiva Software v5.0.2. Unstained cells were used as negative control, and cells with fluorescence higher than the control cells were considered positive. The same gate was used to analyze all cell populations (Fig 5). Liposomes consisting of POPC and those containing 5 mol% DOTAP were almost not taken up by cells (cells positive for RhB < 5%). Liposomes containing 7.5-12.5 mol% DOTAP were taken up in CD14 positive monocytes in a higher degree than the other cell populations (p<0.0001). When 15 mol% DOTAP was incorporated into the membrane more CD19 positive B-lymphocytes started to take up the formulation. Significantly more CD14 cells were positive for RhB than both CD19 cells (p<0.001) and the other three cell populations (p<0.0001). At 20 mol% DOTAP the same amount of CD19 cells were positive for RhB as CD14 cells, whereas significantly less CD3, CD15 and CD56 positive cells were positive for RhB. At 50 mol% DOTAP 85-100% of all cell populations had taken up liposomes. This indicates that the optimal DOTAP contend in order to specific target CD14 positive monocytes in the blood lies between 7.5-12.5 mol% DOTAP.

### EXAMPLE 6

### Liposome targeting to monocytes dependent on freshness of blood

To be able to study liposome uptake in human blood cells an *in vitro* model has to be used. All experiments are conducted on whole blood immediately after the blood is drawn from the donors (within 10 minutes), since fresh blood probably most closely resembles *in vivo* conditions. To investigate the importance of this aspect of the *in vitro* conditions the blood were left for 2h and 4h at 37°C with rotation before the liposome uptake study was performed (Fig. 6). Whole blood was obtained from healthy volunteers as described in example 3. Briefly, 10 µl liposome preparations (formulation 3 and 8 in fig. 1A) were added to 200 µl fresh whole blood in 1.5 ml eppendorf tubes after 0h, 2h and 4h incubation of the blood at 37°C with rotation. Samples were incubated for 1 h at 37°C with rotation. Red blood cells (RBC) were lysed and cells were prepared for FACS analysis as described in example 3. Data was analyzed by BD FACSDiva Software v5.0.2. Unstained cells were used as negative control, and cells with fluorescence higher than the control cells were considered positive. The same gate was used to analyze all cell populations. At t=0h, the uptake was as previously described (example 3, formulation 3 and 8), with significantly higher uptake in CD14 positive cells as compared with the four other populations. After 2h, the percentage of CD14 positive monocytes that had taken up 10% DOTAP decreases significantly compared with CD14 positive cells at t=0h and at the same time, significantly more CD19 positive B-lymphocytes took up liposomes compared with CD19 cells at t=0. At t=2h and t=4h there are no significant difference between uptake in CD14 and CD19 positive cells. The uptake in CD14 monocytes further decreased at t=4h. Together these results demonstrate the importance of the blood being fresh for the *in vitro* conditions to mimic *in vivo* conditions.

### EXAMPLE 7

### Cytotoxic activity of cationic liposomes on the monocytic cell line THP1

A toxicity assay was performed to examine the potential cytotoxic effect of the cationic liposomes (Fig. 7). Liposome cytotoxicity was measured on the human monocytic cell line THP-1 using the formulations from figure 5A (composition 3 + 6, 8, 10, 11, 12 in Fig. 1A). Liposome preparations (10 µl) were added to 200 µl cell suspension (10⁶ cells/ml) in 1.5 ml eppendorf tubes. The cells were incubated with different concentrations (0, 5, 50, 250 and 500 µM) of liposomes for 1 h at 37°C with rotation before washing twice with fresh media. The uptake by THP-1 cells was immediately analyzed by FACS (Fig. 7A). After 24h incubation in fresh media induced apoptosis were measured by analyzing annexin V binding to the cells. Annexin V has specific affinity for phosphatidyleserine that soon after induction of apoptosis is translocated from the inner leaflet of the plasmamambrane to the cell surface (G. Koopman, et al.,Blood, 84 (5): 1415-1420, Sep 1994). Cells were incubated for 24h at 37°C in a humidified air atmosphere in the presence of 5 % CO2 in 96 well plates. 10⁻⁶ M (0.26g/µl) staurosporine was added to one well with cells and used as positive control for apoptosis. Cells were harvested and washed twice with ice cold PBS and re-suspended in binding buffer at a concentration of 10⁶ cells/ml. A total of 100 µl of cell suspension was added 1 µl of Annexin V, mixed gently and incubated at RT for 15 min in dark and analyzed immediately by FACS. There was no significant cytotoxicity of the cationic liposomes containing up to 20 mol% DOTAP compared to untreated cells (Fig. 7B and 7C). At 50 mol% DOTAP the liposomes were toxic to cells, when they were present in high concentrations (250 and 500 µM) detected by significant higher amount of bound annexin V when compared to both the control and the other formulations (P<0.001). A proliferation assay was also conducted on the cells after 24h incubation to analyze the viability of liposome treated cells. Liposome induced cytotoxicity was assessed based on the ability of the cells to convert a tetrazolium reagent, 2,3-bis (2-methoxy-4-nitro-5-sulfophenyl)-5-[(phenylamino)carbonyl]-2H-tetrazolium hydroxide (XTT), into a water-soluble formazan product. Liposome preparations (10 µl) were added to 200 µl cell suspension (10⁶ cells/ml) in 1.5 ml eppendorf tubes. Samples were incubated for 1h at 37°C with rotation. 105 cells were seeded/well in 100 µl culture medium on a 96-well microtitre plate and incubated for 24h at 37°C. XTT labelling mixture (20 µl) was added and cells were further incubated for 4h. The absorbance of the samples was measured at 450 nm subtracted the absorbance at 690 nm as recommended by the manufacturer using an Infinite 200 microplate reader (Tecan, Männedorf, Switzerland). As with annexin V binding liposomes containing 50 mol% DOTAP were toxic to cells at high concentrations (250 and 500 µM), detected by decreased amount of converted XTT.

### EXAM PLE 8

### Test of specific liposomal uptake by monocytes in animal model

RhB labeled liposomes composed of POPC (100) (negative control) and POPC: DOTAP (90: 10) with inclusion of 0,2-0,5 mol % DOPE-rhodamine B and/or tritium labeled POPC (1-2 mol %) are injected into the tail vein of a rodent (e.g. rat or rabbit). After 5 min, 15 min and 1h, blood samples are drawn, RBCs are lysed and the uptake of liposomes in the different cell populations are analyzed by FACS by detection of rhodamine B, as described in example 3. Total amounts of liposomes accumulated in organs like liver, spleen, heart and lung are analyzed by sacrificing the animals, draw a large blood sample, removal of the organs, homogenization of the tissues and detection of the amount of tritium in these organs. It is expected that organs rich in immune cells like blood, liver and spleen will show relatively high tritium levels compared to e.g. heart.

### EXAMPLE 9

### Analyses of therapeutic effect of monocyte targeting for treatment of sepsis

Sepsis is a potentially life-threatening condition where bacteria or their toxic product are spread by the blood. The immune system may injure organs far from the original infection. RhB labeled liposomes composed of glucocorticoids, e.g. dexamethasone phosphate or methyl prednisolone succinate which can both be actively loaded (Avnir et al., PLoS ONE, Oct 2011, vol 6, issue 10, e25721) into the liposome consisting of POPC: DOTAP (90: 10) are injected into the tail vein of a rat model of acute inflammation. Relevant models of experimental sepsis may include the cecal ligation and puncture model (CLP), or an LPS induced experimental model. Preferably, the glucocorticoid liposomes are injected at the day of initiation of the model, or the subsequent days, either once or with a daily intravenous injection. At certain intervals, markers of inflammation are measured in the blood, typically cytokines like TNF-alpha and IL-6 are monitored in the blood to measure the effect of monocyte suppression.

### EXAMPLE 10

### Analyses of therapeutic effect of monocyte targeting for treatment of Listeria monocytogenes infection

Listeriosis is a serious infection caused by the bacterium Listeria monocytogenes. The bacteria grow and divide intracellular in the cells of the blood (Stavru F. et al., Immunol Rev, 240 (1): 160-184, Mar 2011). Listeria monocytogenes is found in all cell types of the blood as they use InlA and IlnB to enter non-phagocytic cell types. Ampicillin is encapsulated in RhB labeled liposomes composed of POPC: DOTAP (90: 10) and injected into the tail vein of a rat model of *L. monocytogenes.* The number of viable *L. monocytogenes* cells recovered from the liver, spleen and blood are used as a parameter of therapeutic efficacy for treatment of the *L. monocytogenes infection.*

### EXAMPLE 11

### Analyses of therapeutic effect of monocyte targeting for treatment of atherosclerosis

Atherosclerosis is a condition where an artery wall thickens as a result of endothelial dysfunction, which leads to subendothelial accumulation of lipoproteins and adhesion of leucocytes, such as monocytes and T-lymhpocytes. A crucial step in development of the disease is differentiation of monocytes into machrophages in the damaged endothelium and subsequent ingestion of lipids turning them into foam cells. Normal and atherogenic mice are prepared by the method of Yamaguchi et al. (1993). RhB labeled liposomes composed of e.g. dexamethasone phosphate or methyl prednisolone succinate incorporated into POPC:DOTAP (90: 10) are injected into the tail vein. The formation of atherosclerotic lesions in atherogenic mice is checked by monitoring the aortic cholesterol ester (CE) levels.

### EXAMPLE 12

### Treatment of a mammal with Arthritis using immunosuppressive liposomes (non-antigen specific)

Arthritis is an autoimmune condition of the joints involving inflammation with production of proinflammatory cytokines like TNFα and IL-6, presence and migration of inflammatory cells into the joint, and presence of auto-antigen antibodies reacting towards self antigens. Major cell types involved in arthritis development are macrophages and dendritic cells, that differentiate from monocytes that migrates from the periphery into the inflamed joint, where they further promote the inflammatory condition. Thus by suppressing the monocytes in the periphery using immunosuppressive liposomes targeted towards the monocytes, the migrating monocytes may give rise to macrophages and dendritic cells that do not actively promote the inflammatory condition. In order to treat or diminish the arthritic inflammation, liposomes are prepared with POPC: DOTAP (90: 10) as described in example 1, with remote loading of e.g. methyl prednisolone hemisuccinate (MPS) which is a amphipathic weak acid prodrug, MPS is actively loaded into the liposomes using Ca-acetate gradients as described in the method by Avnir et al., Arthritis & Rheumatism, vol 58, no1, 2008, pp119-129. Briefly, a [Ca acetate] liposome -[Ca acetate]medium transmembrane gradient is used as the driving force for the remote loading of the amphipathic weak acid GC prodrugs. Such a gradient is characterized by an excess of the membrane-impermeable Ca²⁻ ions over the membrane-permeable acetic acid. In such liposomes MPS in its protonated (uncharged) form is "pumped" into the slightly alkaline intraliposomal aqueous phase by diffusing through the lipid bilayer. There it loses its proton, becomes negatively charged, and forms a poorly soluble MPS-calcium salt, which precipitates in the intraliposomal aqueous phase. The liposomes are administered to a patient suffering from rheumatoid arthritis in an isotonic buffer, e.g. saline and/or with sucrose. The MPS-liposomes are administered slowly intravenously preferably in one of the larger veins to reduce adherence and uptake by endothelial cells. The MPS-liposomes will thus adhere and associate with peripheral monocytes, which phagocytose the MPS-liposomes. During this process MPS is released from the liposomes and will to a certain extent activate the nuclear glucocorticoid receptor, which will suppress subsequent monocyte activation, once the monocyte approach the inflammatory arthritic milieu. Thus, monocyte differentiation into macrophages and dendritic cells will be suppressed or skewed towards a tolerogenic phenotype, and potentially the migration of monocytes into the inflamed joint will be reduced.

### EXAMPLE 13

### Treatment of a mammal with Arthritis using immunosuppressive liposomes (antigen specific) as an example of a tolerogenic vaccine

To obtain an antigen specific tolerogenic response in an autoimmune condition like rheumatoid arthritis, liposomes are prepared largely as in example 12. However, the liposomes may be loaded with MPS or other tolerance inducing compounds like vitamin D3 or retinoids, or some of their potent analogues, known to induce immune tolerance. Prior to loading of the immunosuppressive compound, liposomes are prepared according to example 1, and an antigen relevant for rheumatoid arthritis is loaded into the liposome, either by active or passive loading, according to the nature of the antigen. Relevant antigens may include collagen type II, human chondrocyte glycoprotein 39, aggrecan (derived from proteoglycan), heat shock proteins, citrullinated filaggrin or other relevant antigens. The liposomes are loaded largely according to Copland et al., Vaccine 21, 2003, pp 883-890. Briefly, POPC and DOTAP (90: 10) are mixed in an orgnic solvent and dried under vacuum, to create a thin lipid film. A certain volume, typically 2 ml, of HEPES buffer pH 7.4 containing the solubilized antigen is added and multilamellar vesicles prepared by shaking. The liposomes are homogenized by extrusion as outlined in example 1. The liposomes are administered intravenously to a patient suffering from rheumatoid arthritis, with the aim of generating monocyte differentiation into dendritic cells that specifically show tolerogenic activity in an antigen specific manner, towards the encapsulated antigen. In order for the targeted monocyte to differentiate into tolerogenic antigen specific dendritic cells, it may be an advantage to include small amounts of an immune stimulating compound, e.g. L18-MDP or a TLR3, TLR7, TLR8 or TLR9 agonist.

### EXAMPLE 14

### Treatment of a mammal with cancer using immunostimulatory liposomes (non-antigen specific)

To obtain an immune stimulatory liposome suitable for cancer treatment, a monocyte targeting liposome is prepared using e.g. POPC and DOTAP (90: 10), with addition of a suitable immune stimulating compound, acting through an intracellular receptor which may be TLR3, 7, 8, 9 or NOD2. In this example the immunostimulatory compound Mifamurtid (2-[(*N*-{(2*R*)-[(2-acetamido-2,3-dideoxy-D-glucopyranos-3-yl)oxy]-propanoyl}-L-alanyl-D-isoglutaminyl-L-alanyl)amino]ethyl (2*R*)-2,3-bis(hexadecanoyloxy)propyl hydrogen phosphate), is formulated together with POPC and DOTAP and dried to a lipid film. This film is hydrated in a buffer suitable for intravenous administration, e.g. saline and glucose. The mifamurtid-liposomes are administered intravenously to a cancer patient suffering from e.g. osteosarcoma, prostate cancer, leukemia, lymphoma or melanoma within a one-two week interval. Monocytes are known to largely migrate to the tumor environment, and are therefore suitable for enhancing the anti-tumor immune response under the right conditions. However, a known phenomenon for tumor development is the production of immune suppressing substances like cytokines and regulatory T-cells, which may be difficult for the intact immune system to overcome. Therefore, the peripheral monocytes that phagocytose the mifamurtid-liposomes are stimulated to boost the immune response, and may show better resistance and overcome the immune tolerance inducing milieu associated with the cancer and tumor area, thereby allowing the mifamurtid-liposome targeted monocytes to stimulate an anti-cancer immune response.

### EXAMPLE 15

### Treatment of a mammal with cancer using immune stimulatory liposomes (antigen specific)

To obtain an antigen specific immune response liposomes are prepared as in example 14, however, the buffer used for hydration of the liposome contains the cancer associated antigen of interest (passive loading). The antigen may also be actively loaded using a method suitable for this. The antigen may be e.g. a MAGE antigen for treatment of melanoma, PSA for treatment of prostate cancer or a third antigen. The antigen together with the immune stimulating compound which may be mifamurtid or a TLR3, -7, -8 and -9 agonist, either alone or in combination, are administered intravenously to a cancer patient corresponding to the loaded antigen. The liposomes are administered to the same patient for a number of times to boost an antigen specific response, preferably with 1-2 weeks interval.

### EXAMPLE 16

### Treatment of a mammal with infectious disease (e.g. Listeria monocytogenes) using immunostimulatory liposomes (non-antigen specific)

Infections with the gram-positive bacteria *Listeria monocytogenes* is one of the most virulent foodborne pathogens, where 20-30 % of clinical infections results in death, exceeding both fatality rates for both *Salmonella* and *Clostridium botulinum.* To obtain a liposome suitable for treatment of an infectious disease like e.g. infections of humans with Listeria monocytogenes, which is an intracellular pathogen that affects monocytes and macrophages, liposomes are prepared using e.g. POPC and DOTAP (90: 10). For Listeriosis treatment, one of three antibiotics is used, including penicillin, ampicillin or trimethoprim-sulfamethoxazole. These antibiotics are formulated in liposomes either by passive loading as mentioned in examples 13, or by active loading similar to the loading of MPS in example 12. The antibiotic-loaded liposomes are prepared in an isotonic buffer, and administered intravenously to a patient suffering from Listeriosis over a longer time. The antibiotic-loaded cationic liposomes will adhere to monocytes, be phagocytosed and release the antibiotic in the intracellular space, in which the bacteria is living and dividing. Thus, the antibiotic may kill intracellular bacteria, block further bacterial division within the monocytes and macrophages, and thus prevent further spread of the infection to the patient.

### EXAMPLE 17

### Vaccine for preventing infectious disease (e.g. influenza virus) using antigen specific immunostimulatory liposomes

To obtain an antigen specific immune response suitable for treatment of an infectious disease like e.g. influenza infections, liposomes are prepared using e.g. POPC and DOTAP (90: 10). Liposomes are prepared as in example 13, however, the buffer used for hydration of the liposome contains an antigen for the influenza virus (passive loading), or loaded actively using a suitable gradient. The antigen together with the immune stimulating compound which may be mifamurtid or a TLR3, -7, -8 and -9 agonist, either alone or in combination, are administered intravenously to a human that wish to reduce the risk of a future influenza infection. The liposomes are administered to the same patient for a number of times to boost an antigen specific response, preferably with 2-3 weeks interval. This vaccine may also be used for subcutaneous or intramuscular administration.

### EXAMPLE 18

### Treatment of a mammal with sepsis using immunosuppressive liposomes

Sepsis is a life-threatening systemic inflammation that involves early production of proinflammatory cytokines, in particular TNFα, IL-1β and IL-6. These cytokines are produced from different cell types, and in particular from monocytes. Suppresion of monocyte production of these proinflammatory cytokines at the early onset and diagnosis of sepsis may reduce production of proinflammatory cytokines, and in particular IL-6, which is a strong predictor for later death of the patient. Immunosuppressive liposomes targeting monocytes may therefore have a strong impact on patients with sepsis, by targeting monocytes and down-regulating their immune activation.

In order to treat or diminish the monocyte activation and systemic inflammation, liposomes are prepared with POPC: DOTAP (90: 10) as described in example 1, with remote loading of e.g. methyl prednisolone hemisuccinate as outlined in example 12.

The liposomes are administered to a patient suffering from sepsis at the stage of diagnosis (typically at hospitalization or shortly later). The MPS-liposomes are administered slowly intravenously, preferably in one of the larger veins to reduce adherence and uptake by endothelial cells. The MPS-liposomes will thus adhere and associate with peripheral monocytes, which phagocytose the MPS-liposomes. During this process MPS is released from the liposomes and will suppress further activation of the monocytes. The patient may be monitored for systemic levels of IL-6, and further treated with the MPS-liposomes if IL-6 exceeds levels at 500-1000 pg/ml.

### REFERENCES

1. Y. Zou, G. Zong, Y. H. Ling, and R. Perez-Soler. Development of cationic liposome formulations for intratracheal gene therapy of early lung cancer. Cancer Gene Ther, 7 (5): 683-696, May 2000.
2. Crispin R Dass and Peter F M Choong. Selective gene delivery for cancer therapy using cationic liposomes: in vivo proof of applicability. J Control Release, 113 (2): 155-163, Jun 2006.
3. Suk Hyun Jung, Soon Hwa Jung, Hasoo Seong, Sun Hang Cho, Kyu-Sung Jeong, and Byung Cheol Shin. Polyethylene glycol-complexed cationic liposome for enhanced cellular uptake and anticancer activity. Int J Pharm, 382 (1-2): 254-261, Dec 2009.
4. Rebecca Anderson, Angels Franch, Margarida Castell, Francisco J Perez-Cano, Rolf Bräuer, Dirk Pohlers, Mieczyslaw Gajda, Alexandros P Siskos, Theodora Katsila, Constantin Tamvakopoulos, Una Rauchhaus, Steffen Panzner, and Raimund W Kinne. Liposomal encapsulation enhances and prolongs the anti-inflammatory effects of water-soluble dexamethasone phosphate in experimental adjuvant arthritis. Arthritis Res Ther, 12 (4): R147, 2010.
5. B. A. t Hart, D. G. Elferink, J. W. Drijfhout, G. Storm, L. van Blooijs, R. E. Bontrop, and R. R. de Vries. Liposome-mediated peptide loading of mhc-dr molecules in vivo. FEBS Lett, 409 (1): 91-95, Jun 1997.
6. Efstathios Karathanasis, Cissy M Geigerman, Charles A Parkos, Leslie Chan, Ravi V Bellamkonda, and David L Jaye. Selective targeting of nanocarriers to neutrophils and monocytes. Ann Biomed Eng, 37 (10): 1984-1992, Oct 2009.
7. K. D. Lee, S. Nir, and D. Papahadjopoulos. Quantitative analysis of liposome-cell interactions in vitro: rate constants of binding and endocytosis with suspension and adherent j774 cells and human monocytes. Biochemistry, 32 (3): 889-899, Jan 1993.
8. C. R. Miller, B. Bondurant, S. D. McLean, K. A. McGovern, and D. F. O'Brien. Liposome-cell interactions in vitro: effect of liposome surface charge on the binding and endocytosis of conventional and sterically stabilized liposomes. Biochemistry, 37 (37): 12875-12883, Sep 1998.
9. Keelung Hong Kyung-Dall Lee and Demitrios Papahadjopoulos. Recognition of liposomes by cells: in vitro binding and endocytosis mediated by specific lipid headgroups and surface charge density. Biochim Biophys Acta, 1103: 185-197, 1992.
10. P. Pires, S. Simões, S. Nir, R. Gaspar, N. Düzgünes, and M. C. Pedroso de Lima. Interaction of cationic liposomes and their dna complexes with monocytic leukemia cells. Biochim Biophys Acta, 1418 (1): 71-84, Apr 1999.
11. Mouldy Sioud and Dag R Sørensen. Cationic liposome-mediated delivery of sirnas in adult mice. Biochem Biophys Res Commun, 312 (4): 1220-1225, Dec 2003.
12. Ikuko Yamane, Makiya Nishikawa, and Yoshinobu Takakura. Cellular uptake and activation characteristics of naked plasmid dna and its cationic liposome complex in human macrophages. Int J Pharm, 305 (1-2): 145-153, Nov 2005.
13. Hongtao Lv, Shubiao Zhang, Bing Wang, Shaohui Cui, and Jie Yan. Toxicity of cationic lipids and cationic polymers in gene delivery. J Control Release, 114 (1): 100-109, Aug 2006.
14. Leuschner F, Dutta P, Gorbatov R, Novobrantseva TI, Donahoe JS, Courties G, Lee KM, Kim JI, Markmann JF, Marinelli B, Panizzi P, Lee WW, Iwamoto Y, Milstein S, Epstein-Barash H, Cantley W, Wong J, Cortez-Retamozo V, Newton A, Love K, Libby P, Pittet MJ, Swirski FK, Koteliansky V, Langer R, Weissleder R, Anderson DG, Nahrendorf M. Therapeutic siRNA silencing in inflammatory monocytes in mice. Nat Biotechnol. 2011 Oct 9;29(11):1005-10.

## Claims

1. A liposome comprising lipids and at least one active ingredient, wherein at least one of the lipids is a cationic lipid; said liposome exhibiting a net positive charge at physiological conditions at which said liposome preferentially adheres to monocytes in freshly drawn blood when compared to adherence to granulocytes, T-lymphocytes, B-lymphocytes and/or NK cells in freshly drawn blood.

2. The liposomes according to claim 1, said liposomes having a relative zeta potential of between 15 and 85 %.

3. The liposomes according to any one of the preceding claims, wherein said liposomes are suitable for intravenous administration.

4. The liposomes according to any one of the preceding claims having a relative zeta potential of between 20 and 80%.

5. The liposomes according to any one of the preceding claims having a relative zeta potential of between 25 and 75%.

6. The liposomes according to any one of the preceding claims, wherein the liposomes comprise less than 20%, such as less than 10%, preferably less than 5%, more preferred less than 2% cholesterol, most preferred substantially no cholesterol.

7. The liposomes according to any one of the preceding claims, wherein at least part of the lipids are selected from the group consisting of phospholipids, sterols and sterol derivatives.

8. The liposomes according to any of the preceding claims, where the lipid comprises or constitutes a member selected from the group consisting of phosphatidylcholine (PC), phosphatidylethanolamine (PE), phosphatidylserine (PS), phosphatidylglycerol (PG), phosphatidylinositol (PI), phosphatidic acid (PA), DPG (bisphosphatidyl glycerol), PEOH (phosphatidyl alcohol), cholesterol, ergosterol and lanosterol.

9. The liposomes according to any one of the preceding claims, wherein the phosphatidylcholines are selected from the group consisting of 1,2-dioleoyl-phosphatidylcholine, 1,2-dipalmitoyl-phosphatidylcholine, 1,2-dimyristoyl-phosphatidylcholine, 1,2-distearoyl-phosphatidylcholine, 1-oleoyl-2-palmitoyl-phosphatidylcholine, 1-oleoyl-2-stearoyl-phosphatidylcholine, 1-palmitoyl-2-oleoyl-phosphatidylcholine and 1-stearoyl-2-oleoyl-phosphatidylcholine.

10. The liposomes according to any one of the preceding claims, wherein the the phosphatidylcholines are selected from the group consisting of 1,2-dioleoyl-phosphatidylcholine, 1,2-dipalmitoyl-phosphatidylcholine, 1,2-dimyristoyl-phosphatidylcholine, 1,2-distearoyl-phosphatidylcholine, and 1-palmitoyl-2-oleoyl-phosphatidylcholine, preferably 1,2-dioleoyl-phosphatidylcholine.

11. The liposomes according to any one of claims 1-8, wherein the phosphatidylethanolamines are selected from the group consisting of 1,2-dioleoyl-phosphatidylethanolamine, 1,2-dipalmitoyl-phosphatidylethanolamine, 1,2-dimyristoyl-phosphatidylethanolamine, 1,2-distearoyl-phosphatidylethanolamine, 1-oleoyl-2-palmitoyl-phosphatidylethanolamine, 1-oleoyl-2-stearoyl-phosphatidylethanolamine, 1-palmitoyl-2-oleoyl-phosphatidylethanolamine, 1-stearoyl-2-oleoyl-phosphatidylethanolamine and N-succinyl-dioleoyl-phosphatidylethanolamine; the phosphatidylserines are selected from the group consisting 1,2-dioleoyl-phosphatidylserine, 1,2-dipalmitoyl-phosphatidylserine, 1,2-dimyristoyl-phosphatidylserine, 1,2-distearoyl-phosphatidylserine, 1-oleoyl-2-palmitoyl-phosphatidylserine, 1-oleoyl-2-stearoyl-phosphatidylserine, 1-palmitoyl-2-oleoyl-phosphatidylserine and 1-stearoyl-2-oleoyl-phosphatidylserine; the phosphatidylglycerols are selected from the group consisting 1,2-dioleoyl-phosphatidylglycerol, 1,2-dipalmitoyl-phosphatidylglycerol, 1,2-dimyristoyl-phosphatidylglycerol, 1,2-distearoyl-phosphatidylglycerol, 1-oleoyl-2-palmitoyl-phosphatidylglycerol, 1-oleoyl-2-stearoyl-phosphatidylglycerol, 1-palmitoyl-2-oleoyl-phosphatidylglycerol and 1-stearoyl-2-oleoyl-phosphatidylglycerol; and the phosphatidic acids are selected from the group consisting of di-palmitoyl-glycerophosphatidic acid, di-stearoyl-glycerophosphatidic acid, di-myrostoyl-glycerophosphatidic acid, di-oleoyl-glycerophosphatidic acid, palmitoyl-oleoyl-glycerophosphatidic acid.

12. The liposomes according to any of the preceding claims, wherein at least part of the lipids is a cationic lipid.

13. The liposomes according to claim 12, wherein the cationic lipids are selected from the group consisting of stearylamine (SA), lauryltrimethylammonium bromide; cetyltrimethylammonium bromide, myristyl trimethylammonium bromide, dimethyldioctadecylammonium bromide (DDAB), 3β-[N-(N',N'-dimethylaminoethane)-carbamoyl]cholesterol (DC-Cholesterol), 1,2-ditetradecanoyl-3-trimethylammonium-propane (DMTAP), 1,2-dioctadecanoyl-3-trimethylammonium-propane (DOTAP) and DOTAP derivatives such as 1,2-di-(9Z-octadecenoyl)-3-trimethylammonium-propane and 1,2-dihexadecanoyl-3-trimethylammonium-propane, 1,2-di-(9Z-octadecenoyl)-3-dimethylammonium-propane (DODAP) and DODAP derivatives such as 1,2-ditetradecanoyl-3-dimethylammonium-propane, 1,2-dihexadecanoyl-3-dimethylammonium-propane, and 1,2-dioctadecanoyl-3-dimethylammonium-propane, 1,2-di-O-octadecenyl-3-trimethylammonium propane (DOTMA), 1,2-dioleoyl-c-(4'-trimethylammonium)-butanoyl-sn-glycerol (DOTB), dioctadecylamide-glycylspermine, SAINT-2, polycationic lipid 2,3-dioleyloxy-N-[2(spermine-carboxamido)ethyl]-N,N-dimethyl-1-propanaminiumtrifluoroacetate (DOSPA), and GL67TM.

14. The liposomes according to claim 13, wherein the cationic lipids are selected from the group consisting of stearylamine (SA), 1,2-dioctadecanoyl-3-trimethylammonium-propane (DOTAP) and 1,2-di-(9Z-octadecenoyl)-3-dimethylammonium-propane (DODAP), preferably 1,2-dioctadecanoyl-3-trimethylammonium-propane (DOTAP).

15. The liposomes according to any one of the preceding claims, wherein at least part of the lipids is a cationic lipopeptide selected from the group consisting of a lipid polyarginine conjugate, a lipid TAT conjugate, a lipid polylysine conjugate, or a cationic lipopolysaccharide or lipopolysaccharide such as a lipid chitosan conjugate.

16. The liposomes according to any one of the preceding claims, wherein the liposomes comprise 0.5-50%, preferably 1-20%, such as 3-15, such as 7.5-12.5% (mol/mol) cationic lipids.

17. The liposomes according to any one of the preceding claims, wherein the alkyl chains of the lipids are C8-C24, preferably C10-C22, more preferred C12-C20, preferably C14-C18, most preferred C16-C18 saturated chains or unsaturated chains, preferably saturated chains.

18. The liposomes according to any one of the preceding claims, wherein at least one liposome is a Large Unilamellar Vesicle (LUV).

19. The liposomes according to any one of the preceding claims, wherein the liposomes have a diameter of 40-2000 nm, preferably 80-1000 nm, more preferred 100-500 nm, preferred 50-200 nm, more preferred 100-150 nm, preferably 100-400 nm.

20. The liposomes according to any one of the preceding claims, wherein said at least one active ingredient is an immuno stimulating compound which is a ligand for intracellular proteins and/or receptors.

21. The liposomes according to claim 20, wherein said intracellular proteins and /or receptors are selected from the group consisting of TLR3, TLR7, TLR8, TLR9 , NOD1, NOD2, NOD5, NALP1, NALP2, NALP3, NALP12, NALP14, IPAF, NAIP, CIITA, RIG-I, MDA5, and LGP2, preferably selected from TLR3, TLR7, TLR8, TLR9 , and NOD2, more preferably TLR7.

22. The liposomes according to claim 21, wherein said at least one active ingredient is an immuno stimulating compound selected from the group consisting of polyinosinic:polycytidylic acid (poly I:C), Polyadenylic-polyuridylic acid (poly A:U), poly I:C-poly-L-lysine (poly-ICLC), poly-ICR, CL264, *N*-palmitoyl-*S*-[2,3-bis(palmitoyloxy)-(2*R,S*)-propyl]- (R)-cysteine-(S)serine-(S)lysine 4 (Pam₃Cys), Monophosphoryl lipid A (MPLA) and other lipopolysaccharides, alpha-galactosylceremaide (αGC), Propirimine, Imiquimod (R837), resiquimod (R848), Gardiquimod, R850 (3M Pharma), R851 (3M Pharma), 852A (3M Pharma), S-27610, 3M-002 (CL075), 3M-003, 3M-005, 3M-006, 3M-007, 3M-012, 3M-13, 3M-031,3M-854 (3M Pharma), CL097, CL264, IC-31, Loxoribine and other imidazoquinolines, ssPolyU, sotirimod (3M Pharma), Isatoribine (Anadys), ANA975 (Anadys/Novartis), SM360320 (Sumitomo), R1354 (Coley Pharmaceuticals) single stranded or double stranded RNA, ORN 02 (5'-UUAUUAUUAUUAUUAUUAUU-3'), ORN 06 5'-UUGUUGUUGUUGUUGUUGUU-3', CpG-ODN DSLIM (Mologen), AVE 0675 (Coley Pharmaceuticals), CpG B oligodeoxynucleotide (ODN) 1018 (Dynavax Technologies ), AZD 1419 (Dynavax), ODN 1982, CpG B ODN 2006 (Coley Pharmaceuticals), IMO 2125 (Idera Pharma), CpG A ODN 2216, CpG A ODN 2336, CpG 2395, CpG ODN 7909 (Coley Pharmaceuticals), CpG 10101 (Coley Pharmaceuticals), CpG ODN AVE0675 (Coley Pharmaceuticals), CpG ODN HYB2093 (Idera Pharmaceuticals/Novartis), CpG ODN HYB2055 (Idera Pharmaceuticals), CpG-ODN IMO-2125 (Idera Pharmaceuticals), CpG C ODN M362, Tolamba (Amb a1 ragweed allergen with covalently linked CpG B class ODN 1018)(Dynavax Technologies), Heplisav (Dynavax Technologies), 10181SS (Dynavax Technologies), IM02055 (Idera Pharmaceuticals), IRS954 (Dynavax Technologies), (flagellin, muramyl dipeptide, saponins such as QS21, Leishmania elongation factor, SB-AS4, threonyl-muramyl dipeptide, L18-MDP, mifamurtid, and OM-174.

23. The liposomes according to claim 22, wherein said at least one active ingredient is an immuno stimulating compound selected from the group consisting of Monophosphoryl lipid A (MPLA), Imiquimod (R837), resiquimod (R848), Gardiquimod, Loxoribine, sotirimod (3M Pharma), Isatoribine (Anadys), SM360320 (Sumitomo), CpG B oligodeoxynucleotide (ODN) 1018 (Dynavax Technologies ), AZD 1419 (Dynavax), ODN 1982, CpG B ODN 2006 (Coley Pharmaceuticals), IMO 2125 (Idera Pharma), CpG A ODN 2216, CpG A ODN 2336, CpG 2395, CpG ODN 7909 (Coley Pharmaceuticals), CpG 10101 (Coley Pharmaceuticals), CpG ODN AVE0675 (Coley Pharmaceuticals), CpG ODN HYB2093 (Idera Pharmaceuticals/Novartis), CpG ODN HYB2055 (Idera Pharmaceuticals), CpG-ODN IMO-2125 (Idera Pharmaceuticals), CpG C ODN M362, Tolamba (Amb a1 ragweed allergen with covalently linked CpG B class ODN 1018)(Dynavax Technologies), Heplisav (Dynavax Technologies), QS21, L18-MDP, mifamurtid, and OM-174.

24. The liposomes according to claim 23, wherein said at least one active ingredient is an immuno stimulating compound selected from the group consisting of Mifamurtid (2-[(*N-*{(2*R*)-[(2-acetamido-2,3-dideoxy-D-glucopyranos-3-yl)oxy]-propanoyl}-L-alanyl-D-isoglutaminyl-L-alanyl)amino]ethyl (2*R*)-2,3-bis(hexadecanoyloxy)propyl hydrogen phosphate), L18MDP (6-O-stearoyl-N-Acetyl-muramyl-L-alanyl-D-isoglutamine), as well as acylated or C₁₂₋₂₀ saturated or unsaturated, ester or ether bound derivatives of the above mentioned immuno stimulatory compounds.

25. The liposomes according to any one of claims 1-19 wherein said at least one active ingredient is an immuno suppressive compound comprising a steroid selected from the group consisting of 21-Acetoxyprefnenolone, Aalclometasone, Algestone, Amicinonide, Beclomethasone, Betamethasone, Betamethasone dipropionate, Betamethasone hemisuccinate, Budesonide, Chloroprednisone, Clobetasol, Blovetasone, Clocortolone, Cloprednol, Corticosterone, Cortisone, Cortivazol, Deflazacort, Desonide, Desoximethasone, dexamethason, Dexamethasone palmitate, Dexamethasone phosphate, Diflorasone, Diflucortolone, Difluprednate, Enoxolone, Fluazacort, Flucloronide, Flumethasone, Flunisolide, Fluocinolone Acetonide, Fludrocortisone, Fluocinonide, Fluocortin Butyl, Fluocortolone, Fluorometholone, Fluperolone, Fluprednidine, Fluprednisolone, Flurandrenolide, Formocortal, Halcinonide, Halomethasone, Halopredone, Hydrocortamate, Hydrocortisone, Limethasone, Mazipredone, Medrysone, Meprednisone, Methyolprednisolone, Methyolprednisolone hemisuccinate, Mometasone Furoate, Paramethasone, Prednicarbate, Prednisolone, Prednisolone palmitate, Prednisolone phosphate, Prednisone, Prednival, Prednylidene, Tixocortal, and Triamcinolone.

26. The liposomes according to claim 25, wherein said at least one active ingredient is an immuno suppressive compound comprising a steroid selected from the group consisting of Betamethasone hemisuccinate, Dexamethasone palmitate, Dexamethasone phosphate, Limethasone, Methylprednisolone hemisuccinate, Prednisolone palmitate, and Prednisolone phosphate.

27. The liposomes according to any one of claims 1-19, wherein said at least one active ingredient is an immuno suppressive compound comprising a small molecule inhibitor acting on intracellular targets selected from the group consisting of c-Fms, PDGFRα, Abl, PDGFRβ, NFκB, IκB, JAK1, JAK2, JAK3, GSK3, p38 MAPK, JNK, KIT, EGFR, ERBB2, ERBB4, VEGFR1, VEGFR2, VEGFR3, FLT3, PKCβ, RAF1, CDK1, CDK2, CDK4, NLRP3, IRF3, STAT1, STAT2, STAT3, STAT4, STAT5, STAT6, Hsp90, Hsp70, PI3K, mTOR, AKT, DNA-PK, ATM, AMPK, PDK-1, S6 kinase, RIP2, TRIF, MYD88, TAK1.

28. The liposomes according to claim 27, wherein said at least one active ingredient is an immuno suppressive compound comprising a small molecule inhibitor acting on intracellular targets selected from the group consisting of indomethacin, CLI-095 (C₁₅H₁₇ClFNO₄S, CAS#243984-11-4), Bay11-7082 (C₁₀H₉NO₂S, CAS#19542-67-7), Triptolide (PG 490), CGP53716, SU9518, PD166326, Celastrol, Tripterin, BIRB-796 (Doramapimod), SB 203580, SB202190, VX-702, NVP-BEZ235, GDC-0980 (RG7422), Ridaforolimus (Deforolimus, AP23573), Nilotinib (Tasigna,AMN107), Sorafenib tosylate (Nexavar), Dasatinib (Sprycel, BMS-354825), MLN518 (CT53518), Vatalanib (PTK787 / ZK222584), OSI-930, AZD2171, Pazopanib (Votrient), IPI-504 (retaspimycin), Deforolimus (Ridaforolimus), GDC-0980 (RG7422, C₂₃H₃₀N₈O₃S, CAS#1032754-93-0), Palomid 529 (C₂₄H₂₂O₆ CAS#914913-88-5), Imatinib mesylate (Gleevec/Glivec), Everolimus (Afinitor, RAD001), Sirolimus (Rapamune, rapamycin), Temsirolimus (Torisel, CCI-779), Bortezomib (Velcade), Gefitinib (Iressa), Canertinib (CI-1033, CAS# 267243-28-7, C₂₄H₂₅ClFN₅O₃), Erlotinib hydrochloride (Tarceva), Pelitinib (EKB-569) (C₂₄H₂₃ClFN₅O₂, CAS#257933-82-7), Vandetanib (Zactima, ZD6474, C₂₂H₂₄BrFN₄O₂, CAS No.: 443913-73-3), Sunitinib (Sutent, SU-11248, C₂₂H₂₇FN₄O₂.C₄H₆O₅, CAS No.: 341031-54-7), Tandutinib (MLN518), C₃₁H₄₂N₆O₄, CAS No.: 387867-13-2, Roscovitine (Seliciclib), C₁₉H₂₆N₆O, CAS No.: 186692-46-6.

29. The liposomes according to any one of claims 1-19 wherein said at least one active ingredient is an immuno tolerance inducing compound selected from the group consisting of vitamin D3 (1,25-dihydroxyvitamin D₃) and retinoic acid (all-trans and 9-cis retinoic acid) and their related synthetic or natural analogues and Betamethasone hemisuccinate, Dexamethasone palmitate, Dexamethasone phosphate, Limethasone, Methylprednisolone hemisuccinate, Prednisolone palmitate, and Prednisolone phosphate.

30. The liposomes according to any one of claims 1-19, wherein said at least one active ingredient is an anti-infectious compound selected from the group consisting of Rifampicin, dideoxycytidine-5'-triphosphate, Clarithromycin, acyclovir, ciprofloxacin, fusidin, gentamicin, chloramphenicol, levofloxacin, oxytetracyclin, tobramycin, natriumcromoglicat, Amoxicillin, Ampicillin., Pivampicillin, Ertapenem, Meropenem, Doripenem, Cefotaxim, Ceftazidim, Ciprofloxacin, Valaciclovir, efavirenz, emtricitabin, tenofovirdisoproxil, Rilpivirine, penicillin, Trimethoprim-sulfamethoxazole, rifampicin, etambutol, isoniazid, pyrazinamide, voriconazole, amphotericin B, caspofungin, flucytosine, itraconazole, doxycyclin, sulfonamides, and sulfamethoxazole.

31. The liposomes according to any one of the preceding claims, further comprising at least one antigen as active ingredient.

32. The liposomes according to claim 31, wherein said at least one antigen is selected from the group consisting of a cancer antigen, a self- or autoimmune antigen, a microbial antigen, an allergen, or an environmental antigen.

33. A lipid-based delivery system for targeting monocytes in fresh blood, said system providing delivery to and release of at least one active ingredient to the targeted monocyte, said system comprising:
(I) lipids comprising at least one cationic lipid; and
(II) at least one active ingredient;
said lipids allowing the formation of liposomes, said liposomes comprising at least part of said at least one active ingredient; said liposome exhibiting a net positive charge at physiological conditions at which said liposome preferentially adheres to monocytes in freshly drawn blood when compared to adherence to granulocytes, T-lymphocytes, B-lymphocytes and/or NK cells in freshly drawn blood.

34. A pharmaceutical formulation suitable for intravenous administration of at least one active ingredient, said pharmaceutical formulation comprising liposomes, said liposomes comprising at least part of said at least one active ingredient, and at least part of said liposomes are liposomes according to any one of claims 1-32.

35. The liposomes according to any one of claims 1-32, the lipid delivery system according to claim 33 or the pharmaceutical composition according to claim 34, for use as a pharmaceutical.

36. The liposomes according to any one of claims 1-32, the lipid delivery system according to claim 33 or the pharmaceutical composition according to claim 34 for use in a monocytic associated prophylaxis, treatment or amelioration.

37. The liposomes according to any one of claims 1-32, the lipid delivery system according to claim 33 or the pharmaceutical composition according to claim 34 for use in the prophylaxis, treatment or amelioration of cancer, an infectious disease, an inflammatory condition or disease, an autoimmune disease or allergy.

38. A use of the liposomes according to any one of claims 1-32 the lipid delivery system according to claim 33 or the pharmaceutical composition according to claim 34 for use in a monocytic associated prophylaxis, treatment or amelioration.

39. A use of the liposomes according to any one of claims 1-32, the lipid delivery system according to claim 33 or the pharmaceutical composition according to claim 34 for use in the prophylaxis, treatment or amelioration of cancer, an infectious disease, an inflammatory condition or disease, an autoimmune disease or allergy.

40. A method for in vitro activation/inhibition of monocytes, comprising the steps:
i) Providing fresh blood;
ii) Administering a liposome according to any one of claims 1-32, the lipid delivery system according to claim 33 or the pharmaceutical composition according to claim 34 to said fresh blood;
iii) Allowing said liposome, lipid delivery system or pharmaceutical composition to react.

41. A method for in vivo activation/inhibition of monocytes in a mammal, comprising the step of administering a liposome according to any one of claims 1-32, the lipid delivery system according to claim 33 or the pharmaceutical composition according to claim 34 to said mammal in an amount sufficient to activate/inhibit said monocytes.

42. A method for in vivo activation/inhibition of monocytes in a mammal, comprising the steps:
i) Providing fresh blood from a mammal in need thereof;
ii) Administering a liposome according to any one of claims 1-32, the lipid delivery system according to claim 33 or the pharmaceutical composition according to claim 34 to said fresh blood;
iii) Allowing said liposome, lipid delivery system or pharmaceutical composition to react with said fresh blood;
iv) Reintroducing said blood into the circulation of said mammal.

43. A method for prophylactic or therapeutic treatment or amelioration of cancer, an infectious disease, an inflammatory condition or disease, an autoimmune disease or allergy, the method comprising administering to a subject in need thereof an effective amount of a liposome according to any one of claims 1-32, the lipid delivery system according to claim 33 or a pharmaceutical composition according to claim 34.
The method according to claim 43, wherein said cancer is selected from the group consisting of B cell lymphoma, Burkitt's (Non-Hodgkin's) lymphoma, glioma, bladder cancer, biliary cancer, brain cancer, breast cancer, cervical carcinoma, colon carcinoma, colorectal cancer, choriocarcinoma, epithelial cell cancer, Kidney cancer, Hodgkins lymphoma, gastric cancer, hepatocellular cancer, leukemia, lung cancer, melanoma, myeloma, non-small cell lung carcinoma, nasopharyngeal cancer, ovarian cancer, oropharyngeal cancer, prostate cancer, pancreatic cancer, renal cancer, skin cancer, squamous cell cancers of cervix and esophagus, testicular cancer, T cell leukemia and vaginal cancer.

44. The method according to claim 43, wherein said infectious disease is caused by an infection from the group consisting of *E. coli, Staphylococcal, Chlamydia, Streptococcal, Pseudomonas, Clostridium difficile, Legionella, Pnetanococcus. Haemophilus, Klebsiella, Enterobacter, Citrobacter, Neisseria, Meningococcus B, Shigella, Salmonella,* Listeria, *Pasteurella, Streptobacillus, Spirillum, Treponema, Actinomyces, Borrelia, Corynebacterium, Tuberculosis, Norcardia, Gardnerella, Campylobacter, Spirochaeta, Proteus, Bacteriodes, Yersenia pestis, H. pylori, anthrax, HIV, Coronavirus, Herpes simples virus 1, Herpes simplex virus 2, cytomegalovirus, Dengue virus, Ebola virus, hepatitis A virus, hepatitis B virus, hepatitis C virus, hepatitis E virus, human papilloma virus, human Metapneumoniavirus, Epstein Barr virus, rotavirus, adenovirus, influenza virus (universal, H1N1v, H7N1, H9N2), Pneumococcus, Para influenza virus, respiratory syncytial virus (RSV), varicella-zoster virus, small pox, monkey pox, West Nile virus, SARS, candidiasis, ringworm, histoplasmosis, blastomycosis, paracoccidioidomycosis, crytococcosis, aspergillosis, chromomycosis, mycetoma infections, pseudallescheriasis, tinea versicolor, amebiasis, trypanosome cruzi, Fascioliasis, Leishmanlasis, Plasmodium. Onchocerciasis, Paragonimiasis, Trypanosoma brucei, Pneumocystis, Trichomonas viginalis, Taenia, Hymenolepsis, Echinococcus, Schistosomiasis, neurocysticercosis, Necator americanus,* and *Trichuris trichuria.*

45. The method according to claim 43, wherein said inflammatory condition or disease is selected from the group consisting of trauma, burns, surgery, coronary artery disease, coronary atherosclerosis, atherosclerosis, Infarct Inflammation and Repair after Myocardial Infarction, cardiac ischemia-reperfusion injury, myocardial inflammation, myocardial ischemia, cardiovascular disease, brain infarct, hemorrhagic shock, reperfusion lesion, dialysis, shock and/or bacterial or viral infection, extracorporeal membrane oxygenation (ECMO), Shock, systemic inflammatory response syndrome (SIRS), sepsis, multi organ failure, rejection of an implant such as organ and/or prostheses, heart and lung bypass surgery, chronic inflammation, asthma and/or stress related disease in the lung, bacterial infection, gangrene, soft tissue infection, and wound infection.

46. The method according to claim 43, wherein said autoimmune disease is selected from the group consisting of diabetes, diabetes mellitus, arthritis (including rheumatoid arthritis, juvenile rheumatoid arthritis, osteoarthritis, psoriatic arthritis), multiple sclerosis, myasthenia gravis, systemic lupus erythematosis, autoimmune thyroiditis, dermatitis (including atopic dermatitis and eczematous dermatitis), psoriasis, Sjogren's Syndrome, including keratoconjunctivitis sicca secondary to Sjogren's Syndrome, alopecia areata, allergic responses due to arthropod bite reactions, Crohn's disease, aphthous ulcer, iritis, conjunctivitis, keratoconjunctivitis, ulcerative colitis, asthma, allergic asthma, cutaneous lupus erythematosus, scleroderma, vaginitis, proctitis, drug eruptions, leprosy reversal reactions, erythema nodosum leprosum, autoimmune uveitis, allergic encephalomyelitis, acute necrotizing hemorrhagic encephalopathy, idiopathic bilateral progressive sensorineural hearing loss, aplastic anemia, pure red cellanemia, idiopathic thrombocytopenia, polychondritis, Wegener's granulomatosis, chronic active pepatitis, Stevens Johnson syndrome, idiopathic sprue, lichen planus, Crohn's disease, Graves ophtalmopathy, sarcoidosis, primary biliary cirrhosis, uveitis posterior, and interstitial lung fibrosis.

47. The method according to claim 43, wherein said allergy is selected from the group consisting of asthma, allergic cough, allergic rhinitis and conjunctivitis, atopic eczema, dermatitis, drug allergies, urticaria, hives, insect bite allergy, dietary allergy, such as fish, milk, wheat, peanut, apple, nut allergy.
